(19) Europäisches Patentamt
European Patent Office
Office européen des brevets

(11) Veröffentlichungsnummer: **0 297 042 A1**

(12) # EUROPÄISCHE PATENTANMELDUNG

(21) Anmeldenummer: **88810407.2**

(22) Anmeldetag: **14.06.88**

(51) Int. Cl.4: **C 07 D 499/00**
A 61 K 31/43
// C07F7/18, C07F9/65, C07D205/08

(30) Priorität: **23.06.87 CH 2352/87**

(43) Veröffentlichungstag der Anmeldung: **28.12.88 Patentblatt 88/52**

(84) Benannte Vertragsstaaten: **AT BE CH DE ES FR GB GR IT LI LU NL SE**

(71) Anmelder: **CIBA-GEIGY AG**
**Klybeckstrasse 141**
**CH-4002 Basel (CH)**

(72) Erfinder: **Hungerbühler, Ernst, Dr.**
**Zeisigweg 4**
**CH-4310 Rheinfelden (CH)**

**Lang, Marc, Dr.**
**Rue des Ormes 6**
**F-68170 Rixheim (FR)**

**Scartazzini, Riccardo, Dr.**
**Conrad Ferdinand Meyer-Strasse 48**
**CH-4059 Basel (CH)**

Patentansprüche für folgende Vertragsstaaten: ES + GR.

(54) **Substituierte Penem-Verbindungen.**

(57) Verbindungen der Formel

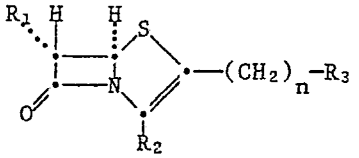

(I),

worin $R_1$ durch Hydroxy oder geschütztes Hydroxy substituiertes Niederalkyl ist, $R_2$ Carboxyl oder funktionell abgewandeltes Carboxyl bedeutet, n eine ganze Zahl von 2 bis 5 ist, $R_3$ Carbamoyloxy oder ein Acyloxy-Rest der Formel -O-C(=O)-Y-N($R_4$,$R_5$) ist, worin $R_4$ Wasserstoff oder Niederalkyl ist, $R_5$ Wasserstoff, Niederalkyl oder Acyl ist und Y Niederalkylen, durch Phenyl substituiertes Niederalkylen oder ein Rest der Formel -$CR_6R_7$-ist, worin $R_6$ für einen über ein Kohlenstoffatom gebundenen organischen Rest steht oder zusammen mit $R_4$ gegebenenfalls durch Oxo oder Hydroxy substituiertes Niederalkylen bedeutet, und $R_7$ Wasserstoff oder Niederalkyl bedeutet, die reinen optischen Isomere von Verbindungen der Formel (I), Mischungen dieser optischen Isomere und Salze solcher Verbindungen besitzen antibiotische Eigenschaften oder können in Verbindungen mit antibiotischen Eigenschaften überführt werden. Die Verbindungen können nach an sich bekannten Verfahren hergestellt werden.

EP 0 297 042 A1

**Beschreibung**

**Substituierte Penem-Verbindungen**

Die Erfindung betrifft 2-Aminoacyloxyalkyl-2-penem-Verbindungen der Formel

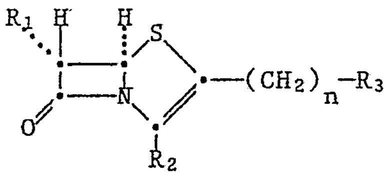

(I),

worin $R_1$ durch Hydroxy oder geschütztes Hydroxy substituiertes Niederalkyl ist, $R_2$ Carboxyl oder funktionell abgewandeltes Carboxyl bedeutet, n eine ganze Zahl von 2 bis 5 ist, $R_3$ Carbamoyloxy oder ein Acyloxy-Rest der Formel -O-C(=O)-Y-N($R_4$,$R_5$) ist, worin $R_4$ Wasserstoff oder Niederalkyl ist, $R_5$ Wasserstoff, Niederalkyl oder Acyl ist und Y Niederalkylen, durch Phenyl substituiertes Niederalkylen oder ein Rest der Formel -$CR_6R_7$- ist, worin $R_6$ für einen über ein Kohlenstoffatom gebundenen organischen Rest steht oder zusammen mit $R_4$ gegebenenfalls durch Oxo oder Hydroxy substituiertes Niederalkylen bedeutet und $R_7$ Wasserstoff oder Niederalkyl bedeutet, die reinen optischen Isomere von Verbindungen der Formel (I), Mischungen dieser optischen Isomere, Salze von Verbindungen der Formel (I), die eine salzbildende Gruppe aufweisen, Verfahren zur Herstellung von Verbindungen der Formel (I), pharmazeutische Präparate, die solche Verbindungen enthalten, und ihre Verwendung zur Herstellung von pharmazeutischen Präparaten oder als pharmakologisch wirksame Verbindungen.

Die vor- und nachstehend verwendeten Definitionen haben im Rahmen der vorliegenden Beschreibung vorzugsweise die folgenden Bedeutungen:

Funktionell abgewandeltes Carboxyl $R_2$ ist insbesondere unter physiologischen Bedingungen spaltbares verestertes Carboxyl oder geschütztes Carboxyl $R_2'$.

Eine unter physiologischen Bedingungen spaltbare (d.h. metabolisierbare) veresterte Carboxylgruppe $R_2$ ist in erster Linie eine Acyloxymethoxycarbonylgruppe oder auch Acylaminomethoxycarbonylgruppe, worin Acyl z.B. der Rest einer organischen Carbonsäure, in erster Linie einer gegebenenfalls, z.B. durch Amino, substituierten Niederalkancarbonsäure oder Arencarbonsäure, z.B. Benzoesäure, ist oder worin Acyloxymethyl den Rest eines Lactons bildet. Solche Gruppen sind z.B. Niederalkanoyloxymethoxycarbonyl, Aminoniederalkanoyloxymethoxycarbonyl, insbesondere $\alpha$-Aminoniederalkanoyloxymethoxycarbonyl, und Niederalkanoylaminomethoxycarbonyl. Weitere unter physiologischen Bedingungen spaltbare veresterte Carboxylgruppen $R_2$ sind z.B. 3-Phthalidyloxycarbonyl, 1-Niederalkoxycarbonyloxyniederalkoxycarbonyl, 1-Niederalkoxyniederalkoxycarbonyl und 2-Oxo-1,3-dioxolen-4-ylmethoxycarbonyl, welches in 5-Stellung des Dioxolenringes gegebenenfalls durch Niederalkyl oder Phenyl substituiert ist.

Eine Acylgruppe $R_5$ hat bis zu 15 Kohlenstoffatome, insbesondere bis zu 7 Kohlenstoffatome, und ist z.B. die Acylgruppe einer Carbonsäure, eines Halbesters der Kohlensäure, einer Carbaminsäure, einer substituierten Carbaminsäure, einer Sulfonsäure, der Amidosulfonsäure oder einer substituierten Amidosulfonsäure. Solche Reste sind beispielsweise Niederalkanoyl, durch Hydroxy, Niederalkoxy, Amino, Halogen, Carboxy und/oder Cyano substituiertes Niederalkanoyl, Benzoyl, durch Amino, Niederalkylamino, Diniederalkylamino, Niederalkanoylamino, Carboxy, Cyano, Nitro, Halogen und/oder Niederalkyl substituiertes Benzoyl, Phenylniederalkanoyl, im Niederalkanoylteil durch Hydroxy oder Amino substituiertes Phenylniederalkanoyl, Niederalkoxycarbonyl, Carbamoyl oder Sulfo. Als Acylreste $R_5$ kommen auch die bei den Aminoschutzgruppen genannten Acylreste in Betracht.

Ein über ein Kohlenstoffatom gebundener organischer Rest $R_6$ hat bis zu 18, bevorzugt bis zu 10 Kohlenstoffatome und ist z.B. ein entsprechender gesättigter oder ungesättigter niederaliphatischer, gesättigter oder ungesättigter cycloaliphatischer, aromatischer oder aromatisch-niederaliphatischer Kohlenwasserstoffrest oder ein heterocyclischer oder heterocyclisch-niederaliphatischer Rest.

Ein entsprechender niederaliphatischer Rest $R_6$ ist z.B. Niederalkyl, ferner auch Niederalkenyl.

Ein entsprechender cycloaliphatischer Rest $R_6$ ist beispielsweise Cycloalkyl oder ungesättigtes Cycloalkyl, wie Cycloalkenyl oder Cycloalkadienyl.

Als aromatischer Kohlenwasserstoffrest ist $R_6$ beispielsweise ein entsprechender monocyclischer oder auch polycyclischer, wie bicyclischer Rest, insbesondere Phenyl.

Bei einem aromatisch-niederaliphatischen Kohlenwasserstoffrest $R_6$ handelt es sich beispielsweise um Phenylniederalkyl.

Ein heterocyclischer Rest $R_6$ ist insbesondere ein entsprechender monocyclischer oder polycyclischer, insbesondere monocyclischer oder bicyclischer Rest, wie ein gegebenenfalls partiell gesättigter monocyclischer 5- oder 6-gliedriger Heteroaryl-Rest mit 1-4 Ringstickstoffatomen und gegebenenfalls einem zusätzlichen Ringheteroatom ausgewählt aus der Gruppe Sauerstoff und Schwefel, z.B. ein entsprechender aza-, diaza-, triaza-, tetraza-, oxa-, oxaza-, oxadiaza-, oxatriaza-, thia-, thiaza-, thiadiaza- oder thiatriaza-cyclischer Rest aromatischen Charakters, oder ein entsprechender Dihydro- oder Tetrahydro-Rest, ferner

gegebenenfalls partiell gesättigte Benzo-, Pyrido-oder Pyrimidoderivate solcher 5- oder 6-gliedrigen Reste. Entsprechende Heteroarylreste $R_6$ sind beispielsweise Pyrrolyl, wie 2- oder 3-Pyrrolyl, Diazolyl, wie Imidazolyl, z.B. 2- oder 4-Imidazolyl, oder Pyrazolyl, z.B. 3- oder 4-Pyrazolyl, Triazolyl, z.B. 1,2,3-Triazol-4-yl oder 1,2,4-Triazol-3-yl, Tetrazolyl, z.B. 1H- oder 2H-Tetrazol-5-yl, Pyridyl, z.B. 2-, 3- oder 4-Pyridyl, Diazinyl, wie Pyrimidyl, z.B. 2-, 4- oder 5-Pyrimidyl, Furyl, z.B. 2- oder 3-Furyl, Thienyl, z.B. 2- oder 3-Thienyl, Oxazolyl, z.B. 2- oder 4-Oxazolyl, Oxadiazolyl, z.B. 1,2,4-Oxadiazol-3-yl oder 1,3,4-Oxadiazol-2-yl, Isoxazolyl, z.B. 3-Isoxazolyl, Thiazolyl, z.B. 2- oder 4-Thiazolyl, Thiadiazolyl, z.B. 1,2,4-Thiadiazol-3-yl oder 1,3,4-Thiadiazol-2-yl, oder Isothiazolyl, z.B. 3- oder 4-Isothiazolyl. Die genannten 5- und 6-gliedrigen Heteroarylreste können auch in partiell gesättigter Form vorliegen. Polycyclische Derivate der genannten Heteroarylreste sind z.B. Indol-3-yl oder Benzimidazol-2-yl.

Heterocyclisch-niederaliphatische Reste $R_6$ sind z.B. Niederalkylreste, die durch einen oder zwei, insbesondere einen, der oben genannten über ein Kohlenstoffatom gebundenen Heterocyclylreste oder durch einen gegebenenfalls partiell gesättigten monocyclischen 5-oder 6-gliedrigen über ein Ringstickstoffatom gebundenen Heteroarylrest, welcher als Ringheteroatome 1 bis 4 Stickstoffatome enthält, substituiert ist. Bei den zuletzt genannten über ein Stickstoffatom gebundenen Resten haldelt es sich beispielsweise um Imidazol-1-yl, Pyrazol-1-yl, 1,2,4-Triazol-1-yl, Tetrazol-1-yl, Tetrazol-2-yl oder 1-Pyridinio.

Die genannten organischen Reste $R_6$ sind unsubstituiert oder können z.B. durch gegebenenfalls veräthertes oder verestertes, inklusive geschütztes Hydroxy, z.B. Hydroxy, Niederalkoxy, Niederalkanoyloxy oder Halogen, gegebenenfalls veräthertes Mercapto, z.B. Mercapto oder Niederalkylthio, Niederalkyl; durch Hydroxy, Niederalkoxy, gegebenenfalls verestertes oder amidiertes Carboxy, wie Carboxy, Niederalkoxycarbonyl oder Carbamoyl, gegebenenfalls N-niederalkyliertes Amino, z.B. Amino, Niederalkylamino oder Diniederalkylamino, Niederalkylenamino, Niederalkanoylamino, gegebenenfalls durch Amino und/oder Carboxy substituiertes Niederalkoxycarbonylamino, Mercapto, Niederalkylthio oder Sulfo substituiertes Niederalkyl; gegebenenfalls substituiertes Amino, z.B. Amino, Niederalkylamino, Diniederalkylamino, Niederalkylenamino, Guanidino, Ureido, Acylamino, wie Niederalkanoylamino, oder gegebenenfalls durch Amino und/oder Carboxy substituiertes Niederalkoxycarbonylamino; gegebenenfalls funktionell abgewandeltes Carboxyl oder Sulfo, z.B. Carboxyl, verestertes Carboxyl, wie Niederalkoxycarbonyl, amidiertes Carboxyl, wie gegebenenfalls substituiertes Carbamoyl, z.B. Carbamoyl oder N-mono- oder N,N-diniederalkyliertes Carbamoyl, Cyano, Sulfo oder Sulfamoyl, gegebenenfalls z.B. durch Niederalkyl, Nitro, Amino, Hydroxy, Niederalkoxy, Carboxy und/oder Halogen substituiertes Phenyl, Nitro, Oxo und/oder Oxido substituiert, wie z.B. mono- oder auch poly-, wie insbesondere mono- oder di-, ferner auch trisubstituiert sein.

In der vorliegenden Beschreibung bedeutet der im Zusammenhang mit Definitionen von Gruppen bzw. Verbindungen verwendete Ausdruck "Nieder", das die entsprechenden Gruppen bzw. Verbindungen, sofern nicht ausdrücklich anders definiert, 1 bis 7, bevorzugt 1 bis 4 Kohlenstoffatome enthalten.

Durch Hydroxy substituiertes Niederalkyl $R_1$ ist insbesondere in α-Stellung zum Penem-Ringgerüst durch Hydroxy substituiertes Niederalkyl mit 1 bis 4 Kohlenstoffatomen und bedeutet z.B. 1-Hydroxyprop-1-yl, 1-Hydroxybut-1-yl und insbesondere Hydroxymethyl oder 1-Hydroxyäthyl.

Niederalkanoyloxymethoxycarbonyl ist z.B. Acetoxymethoxycarbonyl oder Pivaloyloxymethoxycarbonyl, α-Aminoniederalkanoyloxymethoxycarbonyl ist z.B. Glycyloxymethoxycarbonyl, L-Valyloxymethoxycarbonyl oder L-Leucyloxymethoxycarbonyl. Niederalkanoylaminomethoxycarbonyl ist z.B. Acetaminomethoxycarbonyl. 1-Niederalkoxycarbonyloxyniederalkoxycarbonyl ist z.B. Aethoxycarbonyloxymethoxycarbonyl oder 1-Aethoxycarbonyloxyäthoxycarbonyl. 1-Niederalkoxyniederalkoxycarbonyl ist z.B. Methoxymethoxycarbonyl oder 1-Methoxyäthoxycarbonyl. Bei einer 2-Oxo-1,3-dioxolen-4-ylmethoxy-Gruppe, welche in 5-Stellung des Dioxolen-Ringes gegebenenfalls durch Niederalkyl oder Phenyl substituiert ist, handelt es sich vor allem um eine 5-Phenyl-und in erster Linie um eine 5-Methyl-2-oxo-1,3-dioxolen-4-ylmethoxy-Gruppe.

Niederalkyl ist z.B. n-Propyl, Isopropyl, n-Butyl, Isobutyl, sek.-Butyl oder tert.-Butyl, ferner auch n-Pentyl, n-Hexyl oder n-Heptyl, in erster Linie jedoch Methyl oder Aethyl.

Niederalkanoyl ist z.B. Formyl, Acetyl oder Propionyl, während Phenylniederalkanoyl z.B. Phenylacetyl ist.

Niederalkoxy ist z.B. Methoxy, ferner Aethoxy, n-Propyloxy, Isopropyloxy oder n-Butyloxy.

Halogen ist z.B. Fluor, Chlor, Brom oder Jod.

Niederalkylamino ist z.B. Methylamino, Aethylamino, n-Propylamino, Isopropylamino oder n-Butylamino, während Diniederalkylamino z.B. Dimethylamino, Diäthylamino, Di-n-propylamino oder Diisopropylamino bedeutet.

Niederalkanoylamino ist z.B. Formylamino, Acetylamino oder Propionylamino.

Niederalkylen Y ist geradkettiges Niederalkylen mit 1 bis 7 Kohlenstoffatomen, wie Methylen, 1,2-Aethylen, 1,3-Propylen, 1,4-Butylen, 1,5-Pentylen, 1,6-Hexylen oder 1,7-Heptylen, oder verzweigtkettiges Niederalkylen mit 2 bis 7 Kohlenstoffatomen, wie durch Niederalkyl, z.B. Methyl oder Aethyl, mono- oder disubstituiertes geradkettiges Niederalkylen, z.B. Aethyliden, Propyliden, wie 1-Propyliden, 1,2-Propylen, 1,2-Butylen, 1,3-Butylen, 2-Methyl-1,2-propylen, 3-Methyl-1,3-butylen oder 2-Methyl-2,3-butylen, wobei das Kohlenstoffatom 1 der genannten Reste der Carbonylgruppe -C(=O)- oder der Aminogruppe $-N(R_4,R_5)$ benachbart sein kann.

Durch Phenyl substituiertes Niederalkylen Y hat im Niederalkylenteil 2 bis 7 Kohlenstoffatome und ist z.B. 1-Phenyl-1,2-äthylen oder 1-Phenyl-1,3-propylen, wobei das Kohlenstoffatom 1 der genannten Reste der Carbonylgruppe -C(=O)- oder der Aminogruppe $-N(R_4,R_5)$ benachbart sein kann.

Niederalkyl hat insbesondere 2 bis 7 Kohlenstoffatome und ist z.B. Vinyl, Allyl, 2-Methylallyl, n-Propenyl oder

Isopropenyl.

Cycloalkyl enthält vorzugsweise 3 bis 8, in erster Linie 5 oder 6 Ringglieder und ist zum Beispiel Cyclopentyl oder Cyclohexyl, ferner Cyclopropyl sowie Cycloheptyl, während Cycloalkenyl z.B. 5 oder 6 Ringglieder und Cycloalkadienyl z.B. 6 Ringglieder enthält und z.B. 1-, 2- oder 3-Cyclohexenyl, 1- oder 2-Cyclopentenyl oder 1,4-Cyclohexadienyl ist.

Phenylniederalkyl ist z.B. Benzyl, 1-Phenyläthyl oder 2-Phenyläthyl.

Niederalkanoyloxy ist z.B. Formyloxy, Acetyloxy oder Propionyloxy.

Niederalkylthio ist z.B. Methylthio oder Aethylthio, ferner auch n-Propylthio, Isopropylthio oder n-Butylthio.

Niederalkoxycarbonyl ist z.B. Methoxycarbonyl oder Aethoxycarbonyl.

Niederalkylenamino weist insbesondere 4 bis 6 Kohlenstoffatome auf und ist z.B. Pyrrolidino oder Piperidino.

Gegebenenfalls durch Amino und/oder Carboxy substituiertes Niederalkoxycarbonylamino ist z.B. Methoxycarbonylamino, Aethoxycarbonylamino oder Isopropoxycarbonylamino, ferner 2-Amino-2-carboxy-äthoxycarbonylamino.

N-Mono-niederalkyliertes Carbamoyl ist z.B. N-Methyl-, N-Aethyl-oder N-Propylcarbamoyl, während N,N-Di-niederalkyliertes Carbamoyl z.B. N,N-Dimethyl- oder N,N-Diäthylcarbamoyl bedeutet.

Eine gemeinsam von den Resten $R_4$ und $R_6$ gebildete, gegebenenfalls durch Hydroxy oder Oxo substituierte Niederalkylen-Gruppe hat 2 bis 5 Kohlenstoffatome und ist insbesondere 1,3-Propylen, 2-Hydroxy-1,3-propylen oder 1-Oxo-1,3-propylen, worin das Kohlenstoffatom 1 der Propylen-Gruppen dem Rest $-\overset{|}{N}-R_5$ benachbart ist.

Beispiele für niederaliphatische Reste $R_6$ sind z.B. Niederalkyl, z.B. Methyl, Aethyl, Isopropyl, Isobutyl oder sek.-Butyl, oder durch Hydroxy, Niederalkoxy, z.B. Methoxy oder Aethoxy, Niederalkanoyloxy, z.B. Acetyloxy, Halogen, z.B. Chlor oder Brom, Mercapto, Niederalkylthio, z.B. Methylthio, Amino, Niederalkylamino, z.B. Methyl-oder Aethylamino, Diniederalkylamino, z.B. Dimethylamino, Guanidino, Ureido, Niederalkanoylamino, z.B. Acetylamino, durch Amino und Carboxy substituiertes Niederalkoxycarbonylamino, wie Aethoxycarbonylamino, z.B. 2-Amino-2-carboxyäthoxycarbonylamino, Carboxyl, Niederalkoxycarbonyl, z.B. Methoxy- oder Aethoxycarbonyl, Carbamoyl, Sulfo, Sulfamoyl und/oder Oxo substituiertes Niederalkyl, wie insbesondere Methyl, Aethyl, n-Propyl oder n-Butyl. Repräsentative Vertreter solcher niederaliphatischer Reste $R_6$ sind z.B. Methyl, Isopropyl, Isobutyl, sek.-Butyl, Hydroxymethyl, 1- oder 2-Hydroxyäthyl, Mercaptomethyl, 2-Methylthioäthyl, Aminomethyl, 1- oder 2-Aminoäthyl, 3-Aminopropyl, 4-Aminobutyl, 3-Guanidylpropyl, 3-Ureidopropyl, Carboxymethyl, Carbamoylmethyl, 2-Carboxyäthyl, 2-Carbamoyläthyl, Carboxyl oder Niederalkoxycarbonyl, wie Methoxycarbonyl.

Beispiele für cycloaliphatische Reste $R_6$ sind z.B. Cycloalkenyl, z.B. 1-oder 2-Cyclohexenyl, oder insbesondere Cyclohexadienyl, z.B. 1,4-Cyclohexadienyl.

Aromatische Kohlenwasserstoffreste $R_6$ sind z.B. Phenyl oder durch Amino, Niederalkylamino, z.B. Methyl- oder Aethylamino, Diniederalkylamino, z.B. Dimethylamino, Niederalkyl, z.B. Methyl, Aminoniederalkyl, z.B. 2-Aminoäthyl, Niederalkylaminoniederalkyl, z.B. 2-Methylaminoäthyl, durch Amino und Carboxy substituiertes Niederalkoxycarbonylaminoniederalkyl, z.B. 2-(2-Amino-2-carboxy-äthoxycarbonylamino)-äthyl, durch Amino und Carboxy substituiertes Nieder alkoxycarbonylamino, z.B. 2-Amino-2-carboxyäthoxycarbonylamino, Nitro, Hydroxy, Niederalkoxy, z.B. Methoxy oder Aethoxy, Halogen, z.B. Chlor oder Brom, oder Carboxy substituiertes, wie insbesondere mono-, aber auch disubstituiertes Phenyl.

Aromatisch-niederaliphatische Reste $R_6$ sind beispielsweise gegebenenfalls substituiertes Phenylniederalkyl, wie insbesondere Benzyl, worin die Substituenten z.B. Niederalkyl, wie Methyl, Nitro, Hydroxy, Niederalkoxy, z.B. Methoxy oder Aethoxy, Carboxy und/oder Halogen, z.B. Chlor, sind.

Heterocyclische Reste $R_6$ sind z.B. gegebenenfalls z.B. durch Carboxy, Niederalkyl, Aminoniederalkyl oder Amino substituiertes Imidazolyl, z.B. 2- oder 4-Imidazolyl oder 2-Amino-4-imidazolyl, gegebenenfalls z.B. durch Niederalkyl, Amino, Carboxyniederalkyl und/oder Phenyl substituiertes Triazolyl, wie 1H-1,2,3-Triazol-4-yl oder 1H-1,2,4-Triazol-3-yl, z.B. die entsprechenden unsubstituierten Reste, 5-Methyl-oder 5-Amino-1H-1,2,4-triazol-3-yl, gegebenenfalls z.B. durch Niederalkyl, Carboxyniederalkyl, Sulfoniederalkyl, Diniederalkylaminoniederalkyl oder gegebenenfalls Substituenten, wie Halogen, enthaltendes Phenyl substituiertes Tetrazolyl, wie 1H- oder 2H-Tetrazol-5-yl, z.B. 1H-Tetrazol-5-yl, 1-Carboxymethyl-, 1-(2-Carboxyäthyl)-, 1-(2-Sulfoäthyl)-, 1-(2-Dimethylaminoäthyl)- oder 1-Phenyl-1H-tetrazol-5-yl, gegebenenfalls z.B. durch Niederalkyl oder Amino substituiertes Thiazolyl, wie 2-oder 4-Thiazolyl, z.B. 2-Thiazolyl, 2-Amino-4-thiazolyl oder 4,5-Dimethyl-2-thiazolyl, gegebenenfalls z.B. durch Amino, Niederalkyl und/oder Phenyl substituiertes Oxazolyl, wie 2- oder 4-Oxazolyl, z.B. die entsprechenden unsubstituierten Gruppen, ferner 4-Methyl-2-oxazolyl oder 2-Amino-4-oxazolyl, gegebenenfalls z.B. durch Halogen, Niederalkyl oder Aminoniederalkyl substituiertes Furyl oder Thienyl, wie 2- oder 3-Furyl oder 2- oder 3-Thienyl, z.B. die entsprechenden unsubstituierten Reste, 5-Methyl- oder 5-Aminomethyl-2-furyl, oder 5-Aminomethyl-2-thienyl oder gegebenenfalls z.B. durch Hydroxy, Halogen, Niederalkyl, Amino und/oder Oxido substituiertes Pyridyl, wie 2-, 3-oder 4-Pyridyl, z.B. die entsprechenden unsubstituierten Gruppen, 1-Oxido-2-pyridyl oder 4-Chlor-1-oxido-2-pyridyl.

Heterocyclisch-niederaliphatische Reste $R_6$ sind z.B. gegebenenfalls substituierte Heterocyclylmethyl-Reste, wie z.B. gegebenenfalls z.B. durch Niederalkyl substituiertes Imidazol-1-ylmethyl oder -4-ylmethyl, z.B. Imidazol-4-ylmethyl, oder gegebenenfalls z.B. durch Niederalkyl, Carboxyniederalkyl, Sulfoniederalkyl,

Diniederalkylaminoniederalkyl, Amino oder gegebenenfalls durch Halogen substituiertes Phenyl substituiertes Tetrazolylmethyl, wie 1H-Tetrazol-1-ylmethyl, 2H-Tetrazol-2-ylmethyl oder 1H-Tetrazol-5-ylmethyl, z.B. die entsprechenden unsubstituierten Reste, 5-Amino-, 5-Carboxymethyl-, 5-(2-Carboxyäthyl)-, 5-Sulfomethyl-, 5-(2-Dimethylaminoäthyl)- oder 5-Phenyl-1H-tetrazol-1-ylmethyl, 5-Amino-, 5-Carboxymethyl-, 5-Sulfomethyl-, 5-(2-Dimethylaminoäthyl)- oder 5-Phenyl-2H-tetrazol-2-ylmethyl, oder 1-(2-Carboxyäthyl)- oder 1-(2-Dimethylaminoäthyl)-2H-tetrazol-5-ylmethyl, Pyridylmethyl, wie Pyridiniomethyl oder 2-, 3- oder 4-Pyridylmethyl, Furylmethyl, z.B. 2-Furylmethyl, Thienylmethyl, z.B. 2-Thienylmethyl, gegebenenfalls z.B. durch Amino substituiertes Oxazolylmethyl, Thiazolylmethyl oder Thiadiazolylmethyl, z.B. 2-Amino-oxazol-4-ylmethyl, 2-Amino-thiazol-4-ylmethyl oder 5-Amino-1,2,4-thiadiazol-3-ylmethyl, oder Indolylmethyl, z.B. Indol-3-ylmethyl.

Bevorzugt als Rest $R_1$ sind Hydroxymethyl und in erster Linie 1-Hydroxyäthyl.

Bevorzugte unter physiologischen Bedingungen spaltbare veresterte Carboxylgruppen $R_2$ sind z.B. Niederalkanoyloxymethoxycarbonyl, z.B. Acetoxymethoxycarbonyl oder Pivaloyloxymethoxycarbonyl, und 1-Niederalkoxycarbonyloxyniederalkoxycarbonyl, z.B. 1-Aethoxycarbonyloxyäthoxycarbonyl.

In bevorzugten Verbindungen der Formel (I) steht der Rest $R_3$ für Carbamoyloxy oder für den Acyloxyrest einer natürlichen, insbesondere einer genetisch enkodierten Aminosäure, wie z.B. für den Acyloxyrest von Glycin, Alanin, Valin, Leucin, Isoleucin, Prolin, Serin, Threonin, Methionin, Phenylalanin, Tryptophan, Tyrosin, Asparagin, Asparaginsäure, Glutamin, Glutaminsäure, Lysin, Arginin oder Histidin, ferner für den Acyloxyrest einer genetisch nicht kodierten Aminosäure, z.B. einer durch Amino und gegebenenfalls zusätzlich durch Phenyl, Hydroxy und/oder Ureido substituierten Niederalkancarbonsäure mit 3 bis 12 Kohlenstoffatomen oder einer gegebenenfalls durch Oxo oder Hydroxy substituierten Azacyclycarbonsäure mit 5 oder 6 Ringgliedern im Azacyclyl-Ring, z.B. β-Alanin, Homoserin, 2-, 3- oder 4-Aminobuttersäure, 2,3-Diaminopropionsäure, 2,3-Diaminobuttersäure, 5-Aminopentansäure, α- oder β-Aminoisobuttersäure, 3-Amino-3-methyl-buttersäure, 4-Amino-4-methyl-pentansäure, 4-Amino-1-methyl-buttersäure, Aminopivalinsäure, 4-Aminopentansäure, 3-Amino-3-phenyl-propionsäure, Citrullin, Ornithin, Piperidin-3-yl-carbonsäure, Piperidin-4-yl-carbonsäure, 3-Hydroxyprolin und Pyrrolidin-5-on-2-carbonsäure, sowie N-niederalkylierten Derivaten, wie den N-Methyl-Derivaten davon, z.B. Sarcosin. Solche Acyloxyreste können sowohl in der natürlichen, d.h. L-Konfiguration, als auch in der D-Konfiguration oder als Gemisch beider Konfigurationen (DL-Form) vorliegen.

Die in den Verbindungen der Formel I vorhandenen funktionellen Gruppen, wie Hydroxy-, Carboxy-, Amino- oder Sulfogruppen, insbesondere die Hydroxygruppe im Rest $R_1$ und die Carboxylgruppe $R_2$, sind gegebenenfalls durch Schutzgruppen geschützt, die in der Penem-, Penicillin-, Cephalosporin- und Peptidchemie verwendet werden. Solche Schutzgruppen schützen die betreffenden funktionellen Gruppen vor unerwünschten Kondensationsreaktionen, Substitutionsreaktionen und dergleichen während der Synthese der Verbindung der Formel I aus ihren Vorstufen. Solche Schutzgruppen sind leicht, d.h. ohne dass unerwünschte Nebenreaktionen stattfinden, beispielsweise solvolytisch, reduktiv oder auch unter physiologischen Bedingungen, abspaltbar.

Schutzgruppen dieser Art sowie ihre Einführung und Abspaltung sind beispielsweise beschrieben in
J.F.W. McOmie, "Protective Groups in Organic Chemistry", Plenum Press, London, New York, 1973,
T.W. Greene, "Protective Groups in Organic Synthesis", Wiley, New York, 1981,
"The Peptides", Vol. I, Schroeder und Luebke, Academic Press, London, New York, 1965 und
Houben-Weyl, "Methoden der Organischen Chemie", Band 15/1, Georg Thieme Verlag, Stuttgart, 1974.

In Verbindungen der Formel (I) können eine Hydroxygruppe im Rest $R_1$, ferner eine im Rest $R_3$ vorhandene Hydroxygruppe beispielsweise durch Acylreste geschützt sein. Geeignete Acylreste sind z.B. gegebenenfalls durch Halogen substituiertes Niederalkoxycarbonyl, z.B. 2-Bromäthoxycarbonyl oder 2,2,2-Trichloräthoxycarbonyl, Niederalkenyloxycarbonyl, z.B. Allyloxycarbonyl, Niederalkenyloxyoxalyl, z.B. Allyloxyoxalyl, oder gegebenenfalls durch Nitro substituiertes Phenylniederalkoxycarbonyl, z.B. Benzyloxycarbonyl oder 4-Nitrobenzyloxycarbonyl. Weitere geeignete Hydroxyschutzgruppen sind z.B. trisubstituiertes Silyl, wie Triniederalkylsilyl, z.B. Trimethylsilyl, Dimethyl-(2,3-dimethylbut-2-yl)-silyl oder tert.-Butyl-dimethylsilyl. Bevorzugt als Hydroxyschutzgruppe sind Triniederalkylsilyl, durch Halogen substituiertes Niederalkoxycarbonyl und Niederalkenyloxycarbonyl.

Eine Carboxylgruppe $R_2$, ferner auch eine im Rest $R_3$ vorhandene Carboxylgruppe, ist üblicherweise in veresterter Form geschützt, wobei die Estergruppe unter schonenden Bedingungen, z.B. unter schonend reduktiven, wie hydrogenolytischen, oder schonend solvolytischen, wie acidolytischen oder insbesondere basisch oder neutral hydrolytischen Bedingungen, leicht spaltbar ist.

Solche veresterten Carboxylgruppen enthalten als veresternde Gruppen in erster Linie in 1-Stellung verzweigte oder in 1- oder 2-Stellung geeignet substituierte Niederalkylgruppen. Geeignete in veresterter Form vorliegende Carboxylgruppen sind unter anderen gegebenenfalls durch Nitro oder Niederalkoxy, wie Methoxy, substituiertes Benzyloxycarbonyl, z.B. 4-Nitrobenzyloxycarbonyl oder 4-Methoxybenzyloxycarbonyl, Niederalkanoylmethoxycarbonyl, wie Acetonyloxycarbonyl, Halogenniederalkoxycarbonyl, wie 2-Halogenniederalkoxycarbonyl, z.B. 2,2,2-Trichloräthoxycarbonyl oder 2-Bromäthoxycarbonyl, Niederalkenyloxycarbonyl, z.B. Allyloxycarbonyl, oder in 2-Stellung durch Trinieder alkylsilyl substituiertes Aethoxycarbonyl, z.B. 2-Trimethylsilyläthoxycarbonyl oder 2-(Di-n-butyl-methyl-silyl)-äthoxycarbonyl. Bevorzugte geschützte Carboxylgruppen $R_2'$ sind die 4-Nitrobenzyloxycarbonyl-, Niederalkenyloxycarbonyl-, insbesondere Allyloxycarbonyl-, und die in 2-Stellung durch Triniederalkylsilyl, z.B. Trimethylsilyl oder Di-n-butyl-methylsily, substituierte Aethoxycarbonylgruppe.

Eine geschützte Aminogruppe, z.B. im Rest $R_3$, kann beispielsweise in Form einer leicht spaltbaren

Acylaminogruppe oder als Azidogruppe vorliegen. In einer entsprechenden Acylaminogruppe ist Acyl beispielsweise der Acylrest einer organischen Säure mit z.B. bis zu 18 Kohlenstoffatomen, insbesondere einer gegebenenfalls z.B. durch Halogen oder Phenyl, substituierten Niederalkancarbonsäure oder insbesondere eines Kohlensäurehalbesters. Solche Acylgruppen sind beispielsweise Niederalkenyloxycarbonyl, z.B. Allyloxycarbonyl, gegebenenfalls in 1- oder 2-Stellung substituiertes Niederalkoxycarbonyl, wie Niederalkoxycarbonyl, z.B. Methoxycarbonyl, Aethoxycarbonyl oder tert.Butoxycarbonyl, gegebenenfalls substituiertes Benzyloxycarbonyl, z.B. 4-Nitrobenzyloxycarbonyl, oder 2-Halogenniederalkoxycarbonyl, z.B. 2,2,2-Trichloräthoxycarbonyl oder 2-Bromäthoxycarbonyl, oder 2-(trisubstituiertes Silyl)-äthoxycarbonyl, wie 2-Triniederalkylsilyläthoxycarbonyl, z.B. 2-Trimethylsilyläthoxycarbonyl oder 2-(Di-n-butylmethyl-silyl)-äthoxycarbonyl. Bevorzugte geschützte Aminogruppen sind z.B. Azido, Niederalkenyloxycarbonylamino, z.B. Allyloxycarbonylamino, und gegebenenfalls durch Nitro substituiertes Benzyloxycarbonylamino.

Salze von erfindungsgemässen Verbindungen sind in erster Linie pharmazeutisch annehmbare, nicht-toxische Salze von Verbindungen der Formel I. Solche Salze werden beispielsweise von den in Verbindungen der Formel I vorhandenen sauren Gruppen, z.B. Carboxylgruppen, gebildet und sind in erster Linie Metall- oder Ammoniumsalze, wie Alkalimetall- und Erdalkalimetall-, z.B. Natrium-, Kalium-, Magnesium- oder Kalziumsalze, sowie Ammoniumsalze mit Ammoniak oder geeigneten organischen Aminen, wie Niederalkylaminen, z.B. Triäthylamin, Hydroxyniederalkylaminen, z.B. 2-Hydroxyäthylamin, Bis-(2-hydroxyäthyl)-amin oder Tris-(2-hydroxyäthyl)-amin, basischen aliphatischen Estern von Carbonsäuren, z.B. 4-Aminobenzoesäure-2-diäthylaminoäthylester, Niederalkylenaminen, z.B. 1-Aethyl-piperidin, Cycloalkylaminen, z.B. Dicyclohexylamin, oder Benzylaminen, z.B. N,N′-Dibenzyläthylendiamin, Dibenzylamin oder N-Benzyl-β-phenäthylamin. Verbindungen der Formel I mit einer basischen Gruppe, z.B. mit einer Aminogruppe, können Säureadditionssalze, z.B. mit anorganischen Säuren, wie Salzsäure, Schwefelsäure oder Phosphorsäure, oder mit geeigneten organischen Carbon- oder Sulfonsäure, z.B. Essigsäure, Bernsteinsäure, Fumarsäure, Maleinsäure, Weinsäure, Oxalsäure, Zitronensäure, Benzoesäure, Mandelsäure, Aepfelsäure, Ascorbinsäure, Methansulfonsäure oder 4-Toluolsulfonsäure bilden. Verbindungen der Formel I mit einer sauren und mit einer basischen Gruppe können auch in Form von inneren Salze, d.h. in zwitterionischer Form, vorliegen.

Zur Isolierung oder Reinigung können auch pharmazeutisch ungeeignete Salze Verwendung finden. Zur therapeutischen Anwendung gelangen nur die pharmazeutisch annehmbaren, nicht-toxischen Salze, die deshalb bevorzugt sind.

Die Penem-Verbindungen der Formel I können in den Resten $R_1$, und/oder $R_3$ zusätzliche Chiralitätszentren besitzen. Beispielsweise kann 1-Hydroxyäthyl als Substituent $R_1$ in der R-, in der S- oder in der racemischen R,S-Konfiguration vorliegen. In bevorzugten Penem-Verbindungen der Formel I weist ein Rest $R_1$, welcher ein asymmetrisches Kohlenstoffatom besitzt, insbesondere 1-Hydroxyäthyl, die R-Konfiguration auf. Ferner kann beispielsweise das Methin-Kohlenstoffatom im Rest $-CR_6R_7-$ eine asymmetrische Substitution aufweisen und dementsprechend in der R-, S- oder der racemischen R,S-Konfiguration vorliegen. Die Erfindung betrifft demgemäss die reinen Diastereomeren und Mischungen der Diastereomeren von Verbindungen der Formel I, welche im Rest $R_1$ und/oder $R_3$ zusätzliche Chiralitätszentren aufweisen.

Die Verbindungen der Formel I weisen wertvolle pharmakologische Eigenschaften auf oder können als Zwischenprodukte zur Herstellung von solchen Verbindungen mit wertvollen pharmakologischen Eigenschaften verwendet werden. Verbindungen der Formel I, worin $R_1$ durch Hydroxy substituiertes Niederalkyl ist, n und $R_3$ die unter Formel I angegebenen Bedeutungen haben, wobei funktionelle Gruppen in ungeschützter Form vorliegen, und $R_2$ Carboxyl oder eine unter physiologischen Bedingungen spaltbare veresterte Carboxylgruppe darstellt, und pharmakologisch annehmbare Salze von solchen Verbindungen mit salzbildenden Gruppen haben antibakterielle Wirkungen. Beispielsweise sind sie in vitro gegen grampositive und gramnegative Kokken, inklusive Methicillinresistente Staphylokokken, z.B. Staphylococcus aureus, Staphylococcus epidermidis, Streptococcus pyrogenes, Streptococcus pneumoniae und Streptococcus faecalis, und anaerobe Keime, z.B. Bacteroides sp. und Clostridium sp., in minimalen Konzentrationen von ca. 0,01 bis ca. 8 µg/ml und gegen gramnegative Stäbchenbakterien, wie Enterobacteriaceae, Haemophilus influenzae und Pseudomonas sp., in minimalen Konzentrationen von ca. 0,1 bis ca. 64 µg/ml wirksam. In vivo, bei der systemischen Infektion der Maus, z.B. durch Staphylococcus aureus oder Escherichia coli, ergeben sich bei subkutaner oder oraler Applikation erfindungsgemässer Verbindungen $ED_{50}$-Werte von ca. 0,3 bis ca. 15 mg/kg.

Die neuen Verbindungen können als oral oder parenteral applizierbare antibakterielle Antibiotika, z.B. in Form von entsprechenden pharmazeutischen Präparaten, zur Behandlung von Infektionen Verwendung finden.

Verbindungen der Formel I, worin mindestens eine der vorhandenen funktionellen Gruppen in geschützter Form vorliegt, können als Zwischenprodukte zur Herstellung der oben genannten pharmakologisch wirksamen Verbindungen der Formel I verwendet werden.

Die Erfindung betrifft vor allem Verbindungen der Formel I, worin $R_1$ durch Hydroxy oder geschütztes Hydroxy substituiertes Niederalkyl ist; $R_2$ Carboxyl, unter physiologischen Bedingungen spaltbares verestertes Carboxyl oder geschütztes Carboxyl bedeutet; n eine ganze Zahl von 2 bis 5 ist, $R_3$ Carbamoyloxy oder ein Acyloxy-Rest der Formel $-O-C(=O)-Y-N(R_4,R_5)$ ist, worin $R_4$ Wasserstoff oder Niederalkyl ist; $R_5$ Wasserstoff, Niederalkyl, Niederalkanoyl, durch Hydroxy, Niederalkoxy, Amino, Halogen, Carboxy und/oder Cyano substituiertes Niederalkanoyl; Benzoyl, durch Amino, Niederalkylamino, Diniederalkylamino, Niederalkanoylamino, Carboxy, Halogen, Nitro, Cyano und/oder Niederalkyl substituiertes Benzoyl, Phenylniederalkanoyl, im Niederalkanoylteil durch Amino oder Hydroxy substituiertes Phenylniederalkanoyl, Carbamoyl, Sulfo oder eine Aminoschutzgruppe ist und Y geradkettiges Niederalkylen mit 1 bis 7 Kohlenstoffatomen, verzweigtes Niederalkylen mit 2 bis 7 Kohlenstoffatomen, durch Phenyl substituiertes Niederalkylen oder eine Gruppe der Formel $-CR_6R_7-$ ist, worin $R_6$ Niederalkyl, Niederalkenyl, Cycloalkyl, Cycloalkenyl, Cycloalkadienyl, Phenyl, Phenylniederalkyl, einen über ein Ringkohlenstoffatom gebundenen monocyclischen 5-oder 6-gliedrigen gegebenenfalls partiell gesättigten Heteroarylrest mit 1-4 Ringstickstoffatomen und gegebenenfalls einem zusätzlichen Ringheteroatom ausgewählt aus der Gruppe Sauerstoff und Schwefel, z.B. einen entsprechenden aza-, diaza-, triaza-, tetraza-, oxa-, oxaza-, oxadiaza-, oxatriaza-, thia-, thiaza-, thiadiaza- oder thiatriaza-cyclischen Rest aromatischen Charakters, oder einen entsprechenden Dihydro- oder Tetrahydrorest, oder ein gegebenenfalls partiell gesättigtes Benzo-, Pyrido- oder Pyrimido-Derivat eines solchen 5-oder 6-gliedrigen Restes, oder einen durch einen über ein Ringkohlenstoffatom gebundenen monocyclischen 5- oder 6-gliedrigen gegebenenfalls partiell gesättigten Heteroarylrest mit 1-4 Ringstickstoffatomen und gegebenenfalls einem zusätzlichen Ringheteroatom ausgewählt aus der Gruppe Sauerstoff und Schwefel, oder einen durch einen über ein Ringstickstoffatom gebundenen monocyclischen, 5- oder 6-gliedrigen gegebenenfalls partiell gesättigten Heteroarylrest mit 1-4 Ringstickstoffatomen substituierten Niederalkylrest darstellt, welche Reste $R_6$ unsubstituiert oder durch Hydroxy, Niederalkoxy, Niederalkanoyloxy, Halogen, Mercapto, Niederalkylthio, gegebenenfalls durch Hydroxy, Niederalkoxy, Carboxy, Niederalkoxycarbonyl, Carbamoyl, gegebenenfalls N-niederalkyliertes Amino, Niederalkylenamino, Niederalkanoylamino, gegebenenfalls durch Amino und/oder Carboxy substituiertes Niederalkoxycarbonylamino, Mercapto, Niederalkylthio oder Sulfo substituiertes Niederalkyl; Amino, Niederalkylamino, Diniederalkylamino, Niederalkylenamino, Guanidino, Ureido, Niederalkanoylamino, gegebenenfalls durch Amino und/oder Carboxy substituiertes Niederalkoxycarbonylamino, Carboxyl, Niederalkoxycarbonyl, Carbamoyl, N-mono-oder N,N-diniederalkyliertes Carbamoyl, Cyano, Sulfo, Sulfamoyl, gegebenenfalls durch Niederalkyl, Nitro, Amino, Hydroxy, Niederalkoxy, Carboxy und/oder Halogen substituiertes Phenyl, Nitro, Oxo und/oder Oxido substituiert sind; oder worin $R_6$ zusammen mit $R_4$ gegebenenfalls durch Hydroxy oder Oxo substituiertes Niederalkylen bedeutet, und $R_7$ Wasserstoff oder Niederalkyl bedeutet, die reinen optischen Isomere von Verbindungen der Formel I, welche im Rest $R_1$ und/oder $R_3$ Chiralitätszentren besitzen, Mischungen dieser optischen Isomere und Salze von solchen Verbindungen der Formel I, die eine salzbildende Gruppe aufweisen.

Die Erfindung betrifft hauptsächlich Verbindungen der Formel (I), worin $R_1$ Hydroxymethyl oder 1-Hydroxyäthyl ist, $R_2$ Carboxyl oder eine unter physiologischen Bedingungen spaltbare veresterte Carboxylgruppe ist, n 2 oder 3 ist, und $R_3$ für Carbamoyloxy oder für den Acyloxyrest einer genetisch enkodierten Aminosäure oder für den Acyloxyrest von β-Alanin, 2-, 3- oder 4-Aminobuttersäure, α- oder β-Aminoisobuttersäure, 3-Amino-3-methyl-buttersäure, 4-Amino-4-methyl-pentansäure, 4-Aminopentansäure, oder für ein N-Niederalkyl-Derivat eines solchen Acyloxyrestes steht, die reinen Diastereomere von Verbindungen der Formel (I), die im Rest $R_1$ und/oder $R_3$ Chiralitätszentren besitzen, Mischungen solcher Diastereomere, und Salze von solchen Verbindungen der Formel (I), die eine salzbildende Gruppe aufweisen.

Die Erfindung betrifft vor allem Verbindungen der Formel (I), worin $R_1$ Hydroxymethyl oder 1-Hydroxyäthyl ist, $R_2$ Carboxyl oder unter physiologischen Bedingungen spaltbares verestertes Carboxyl bedeutet, n 2 oder 3 ist und $R_3$ für Carbamoyloxy, Glycycloxy, Alanyloxy, β-Alanyloxy, 3-Amino-3-methyl-butyryloxy oder 4-Aminobutyryloxy steht, die reinen Diastereomeren von Verbindungen der Formel (I), worin $R_1$ 1-Hydroxyäthyl ist und/oder die im Rest $R_3$ ein Chiralitätszentrum besitzen, Mischungen solcher Diastereomere, und Salze von solchen Verbindungen der Formel (I), die eine salzbildende Gruppe aufweisen.

Die Erfindung betrifft in erster Linie Verbindungen der Formel I, worin $R_1$ 1-Hydroxyäthyl ist, $R_2$ Carboxyl bedeutet, n 2 ist und $R_3$ für Glycyloxy, β-Alanyloxy, Carbamoyloxy oder 4-Aminobutyryloxy steht, und Salze davon.

Die Erfindung betrifft vor allem die in den Beispielen genannten Verbindungen der Formel I und ihre pharmazeutisch annehmbaren Salze.

Die Verbindungen der vorliegenden Erfindung können nach an sich bekannten Verfahren hergestellt werden.

Die neuen Verbindungen werden z.B. hergestellt, indem man

a) eine Ylid-Verbindung der Formel

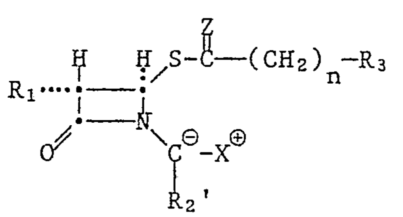

(II),

worin $R_1$, n und $R_3$ die unter Formel I angegebenen Bedeutungen haben, $R_2'$ eine geschützte Carboxylgruppe ist, Z Sauerstoff oder Schwefel bedeutet und $X^{\oplus}$ entweder eine dreifach substituierte Phosphoniogruppe oder eine zweifach veresterte Phosphonogruppe zusammen mit einem Kation

darstellt, ringschliesst, oder

b) eine Verbindung der Formel

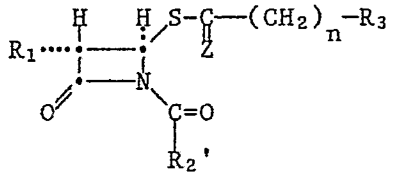

(III),

worin $R_1$, n und $R_3$ die unter Formel I angegebenen Bedeutungen haben, Z Sauerstoff oder Schwefel ist und $R_2'$ eine geschützte Carboxylgruppe ist, mit einer organischen Verbindung des dreiwertigen Phosphors behandelt, oder

c) in einer Verbindung der Formel

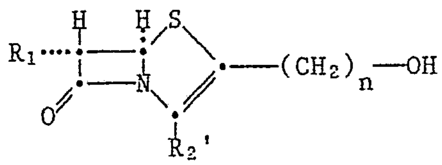

(IA) ,

worin $R_1$ und n die unter Formel I angegebenen Bedeutungen haben und $R_2'$ eine geschützte Carboxylgruppe ist, die Hydroxygruppe durch Acylierung in einen Acyloxyrest $R_3$ überführt, und wenn erwünscht oder notwendig, in einer erhältlichen Verbindung der Formel I eine geschützte funktionelle Gruppe in die freie funktionelle Gruppe überführt, und/oder, wenn erwünscht, in einer erhältlichen Verbindung der Formel I eine freie Carboxylgruppe $R_2$ in eine unter physiologischen Bedingungen spaltbare veresterte Carboxylgruppe überführt, und/oder, wenn erwünscht, in einer erhältlichen Verbindung der Formel I einen Rest $R_3$ in einen anderen Rest $R_3$ überführt, und/oder, wenn erwünscht, ein erhältliches Gemisch von isomeren Verbindungen der Formel I in die einzelnen Isomere auftrennt, und/oder, wenn erwünscht, eine erhältliche Verbindung mit salzbildender Gruppe in ein Salz oder ein erhältliches Salz in die freie Verbindung oder in ein anderes Salz überführt.

In den Ausgangsverbindungen der Formeln II und III sind funktionelle Gruppen, wie freie Hydroxygruppen, z.B. im Rest $R_1$, und freie Aminogruppen vorzugsweise durch konventionelle Schutzgruppen, z.B. durch eine der oben genannten, geschützt.

Cyclisierung der Verbindung der Formel II

Die Gruppe $X^{\oplus}$ im Ausgangsmaterial der Formel II ist eine der bei Wittig-Kondensationsreaktionen gebräuchlichen Phosphonio- oder Phosphonogruppen, insbesondere eine Triaryl-, z.B. Triphenyl-, oder Triniederalkyl-, z.B. Tri-n-butylphosphoniogruppe, oder eine durch Niederalkyl, z.B. Aethyl, zweifach veresterte Phosphonogruppe, wobei das Symbol$X^{\oplus}$ für den Fall der Phosphonogruppe zusätzlich das Kation einer starken Base, insbesondere ein geeignetes Metall-, wie Alkalimetallion, z.B. das Lithium-, Natrium- oder Kaliumion, umfasst. Bevorzugt als Gruppe $X^{\oplus}$ sind einerseits Triphenylphosphonio und andererseits Diäthylphosphono zusammen mit einem Alkalimetall-, z.B. dem Natriumion.

Der Ringschluss kann spontan, d.h. bei der Herstellung der Ausgangsstoffe, oder durch Erwärmen, z.B. in einem Temperaturbereich von etwa 30°C bis 160°C, vorzugsweise von etwa 80°C bis etwa 140°C, erfolgen. Die Reaktion wird vorzugsweise in einem geeigneten inerten Lösungsmittel, wie in einem aliphatischen, cycloaliphatischen oder aromatischen Kohlenwasserstoff, z.B. Cyclohexan, Benzol oder Toluol, einem halogenierten Kohlenwasserstoff, z.B. Methylenchlorid, oder einem Aether, z.B. Diäthyläther, Dioxan oder Tetrahydrofuran, oder in einem Gemisch davon, und, falls notwendig, in einer Inertgas-, z.B. Stickstoffatmosphäre, durchgeführt.

Cyclisierung der Verbindung der Formel III

Eine organische Verbindung des dreiwertigen Phosphors leitet sich z.B. von phosphoriger Säure ab und ist insbesondere ein Ester derselben mit einem Niederalkanol, z.B. Methanol oder Aethanol, und/oder einer gegebenenfalls substituierten aromatischen Hydroxyverbindung, z.B. Phenol oder Brenzcatechin, oder ein Amidester derselben der Formel $P(OR_a)_2N(R_b)_2$, worin $R_a$ und $R_b$ unabhängig voneinander Niederalkyl, z.B. Methyl, oder Aryl, z.B. Phenyl, bedeuten. Bevorzugte Verbindungen des dreiwertigen Phosphors sind Triniederalkylphosphite, z.b. Trimethylphosphit oder Triäthylphosphit.

Die Reaktion wird vorzugsweise in einem inerten Lösungsmittel, wie einem aromatischen Kohlenwasserstoff, z.B. Benzol oder Toluol, einem Aether, z.B. Dioxan oder Tetrahydrofuran, oder einem halogenierten Kohlenwasserstoff, z.B. Methylenchlorid oder Chloroform, bei einer Temperatur von etwa 20° bis 140°C, bevorzugt bei etwa 40° bis etwa 110°C, durchgeführt, wobei man 1 Moläquivalent einer Verbindung der Formel III mit 2 Moläquivalenten der Phosphorverbindung umsetzt. Vorzugsweise legt man die Verbindung der Formel III in einem inerten Lösungsmittel vor und tropft die Phosphorverbindung, vorzugsweise in dem gleichen inerten Lösungsmittel gelöst, über einen längeren Zeitraum, z.B. während eines Zeitraums von 2 bis 4 Stunden, hinzu.

In einer bevorzugten Ausführungsform des Verfahrens stellt man das Ausgangsmaterial der Formel III wie weiter unten angegeben her und setzt es ohne Isolierung aus dem Reaktionsgemisch mit der organischen Verbindung des dreiwertigen Phosphors um, wobei die Endprodukte der Formel I entstehen.

Acylierung der Verbindung der Formel IA

Die Hydroxyverbindungen der Formel IA können durch Acylierung mit einem den Carbamoylrest oder den Rest -C(=O)-Y-N($R_4$,$R_5$) einführenden Acylierungsmittel in die Verbindungen der Formel I überführt werden.

So werden Verbindungen der Formel I, worin $R_3$ Carbamoyloxy ist, beispielsweise hergestellt, indem man die Hydroxylverbindung IA bei Raumtemperatur oder bei leicht erniedrigter oder erhöhter Temperatur, z.B. bei 0° bis 30°C, in einem inerten Lösungsmittel, beispielsweise einem Niederalkyläther oder Methylenchlorid, mit einem den Carbamoylrest einführenden Acylierungsmittel, z.B. Carbamoylchlorid oder Trichloracetylisocyanat, umsetzt. Die bei Verwendung von Trichloracetylisocyanat primär entstehende N-Trichloracetylcarbamoyloxy-Verbindung wird durch Solvolyse mittels eines Niederalkanols, beispielsweise Methanol, gegebenenfalls in Gegenwart von Kieselgel bei Raumtemperatur in die gewünschte Carbamoyloxy-Verbindung überführt.

Andererseits kann man in den Verbindungen der Formel IA die Hydroxygruppe in einen Acyloxy-Rest der Formel -O-C(=O)-Y-N($R_4$,$R_5$) umwandeln. Die Umwandlung der Hydroxygruppe in den Acyloxy-Rest erfolgt beispielsweise durch Umsetzen mit einem den Acylrest einer Carbonsäure der Formel HOOC-Y-N($R_4$,$R_5$)(IV) einführenden Acylierungsmittel.

Ein den Acylrest einer Carbonsäure der Formel IV einführendes Acylierungsmittel ist entweder die Carbonsäure der Formel IV selbst oder ein reaktionsfähiges funktionelles Derivat davon.

Falls eine freie Säure der Formel IV zur Acylierung eingesetzt wird, wird die Reaktion üblicherweise in Gegenwart von geeigneten Kondensationsmitteln, wie Carbodiimiden, beispielsweise N,N′-Dicyclohexylcarbodiimid, geeigneten Carbonylverbindungen, beispielsweise Carbonyldiimidazol, oder 1,2-Oxazoliumverbindungen, wie 2-Aethyl-5-phenyl-1,2-oxazolium-3′-sulfonat oder 2-tert.-Butyl-5-methyl-1,2-oxazoliumperchlorat, oder einer geeigneten Acylaminoverbindung, z.B. 2-Aethoxy-1-äthoxycarbonyl-1,2-dihydro-chinolin, gegebenenfalls in Gegenwart einer Base, wie eines Pyridin-Derivats, z.B. 4-Dimethylaminopyridin, durchgeführt.

Ein reaktionsfähiges funktionelles Derivat einer Carbonsäure der Formel IV ist in erster Linie ein Anhydrid, vorzugsweise ein gemischtes Anhydrid. Ein gemischtes Anhydrid wird beispielsweise durch Kondensation mit einer anderen Säure, z.B. einer anorganischen Säure, z.B. einer Halogenwasserstoffsäure, gebildet und ist beispielsweise das entsprechende Carbonsäurehalogenid, z.B. das Carbonsäurechlorid oder -bromid. Ein gemischtes Anhydrid wird ferner durch Kondensation mit Stickstoffwasserstoffsäure gebildet und ist beispielsweise das Carbonsäureazid. Weitere anorganische Säuren, die sich zur Bildung gemischter Anhydride eignen, sind Phosphor-haltige Säuren, z.B. Phosphorsäure, Diäthylphosphorsäure oder phosphorige Säure, oder Schwefel-haltige Säuren, z.B. Schwefelsäure.

Ein reaktionsfähiges funktionelles Derivat einer Carbonsäure der Formel IV ist ebenfalls ein entsprechender aktivierter Ester, der beispielsweise durch Kondensation mit einem vinylogen Alkohol, d.h. mit einem Enol, z.B. einem vinylogen Niederalkenol, gebildet wird, ein Iminomethylesterhalogenid, z.B. Dimethyliminomethylesterchlorid, hergestellt aus der Carbonsäure der Formel IV und z.B. Dimethyl-(1-chloräthyliden)-iminiumchlorid der Formel $[(CH_3)_2N^{\oplus}=C(Cl)CH_3]Cl^{\ominus}$, das man seinerseits z.B. aus N,N-Dimethylacetamid und Phosgen oder Oxalylchlorid erhalten kann, ein Arylester, z.B. ein durch Halogen, z.B. Chlor, und/oder Nitro substituierter Phenylester, z.B. ein Pentachlor-, 4-Nitrophenyl- oder 2,4-Dinitrophenylester, ein N-heteroaromatischer Ester, z.B. N-Benztriazolester, oder ein N-Diacyliminoester, z.B. ein N-Succinimino- oder N-Phthaliminoester.

Die Acylierung mit einem reaktionsfähigen funktionellen Derivat der Carbonsäure der Formel IV, z.B. mit einem entsprechenden Anydrid, insbesondere einem Säurehalogenid, wird bevorzugt in Anwesenheit eines geeigneten säurebindenden Mittels, beispielsweise einer geeigneten organischen Base, durchgeführt. Eine geeignete organische Base ist beispielsweise ein Amin, z.B. ein tertiäres Amin, wie Triniederalkylamin, z.B. Trimethylamin, Triäthylamin oder Aethyl-diisopropylamin, oder ein N,N-Diniederalkylanilin, z.B. N,N-Dimethylanilin, oder ein cyclisches tertiäres Amin, z.B. ein N-niederalkyliertes Morpholin, z.B. N-Methylmorpholin, oder ist beispielsweise eine Base vom Pyridin-Typ, z.B. Pyridin. Ein geeignetes säurebindendes Mittel ist ferner eine anorganische Base, beispielsweise ein Alkalimetall- oder Erdalkalimetallhydroxid, -carbonat oder -hydrogencarbonat, z.B. Natrium-, Kalium- oder Calciumhydroxid, -carbonat oder -hydrogencarbonat.

Die Acylierung mit einer Carbonsäure der Formel IV oder einem reaktionsfähigen, funktionellen Derivat davon wird insbesondere in einem inerten, vorzugsweise wasserfreien Lösungsmittel oder Lösungsmittelgemisch vorgenommen, beispielsweise in einem Carbonsäureamid, wie einem Formamid, z.B. Dimethylformamid, einem halogenierten Kohlenwasserstoff, z.B. Methylenchlorid, Tetrachlorkohlenstoff oder Chlorbenzol, einem Keton, z.B. Aceton, cyclischen Aether, z.B. Tetrahydrofuran, oder einem Nitril, z.B. Acetonitril, oder in Mischungen davon, wenn notwendig oder erwünscht, bei erniedrigter oder erhöhter Temperatur, z.B. in einem

Temperaturbereich von etwa -40° bis etwa +100°C, bevorzugt von etwa -20° bis etwa +50°C, und, falls erforderlich, in einer Inertgas-, z.B. Stickstoffatmosphäre.

In den Ausgangsverbindungen der Formel IV sind vorhandene funktionelle Gruppen in den Resten $R_4$, $R_5$, und Y, z.B. Carboxyl-, Amino-oder Hydroxylgruppen, durch eine der weiter vorn genannten Schutzgruppen, wie Carboxyl-, Amino- oder Hydroxylschutzgruppen, geschützt.

Bevorzugt werden solche Ausgangsmaterialien der Formeln II, III, IA und IV verwendet, die zu den eingangs als besonders bevorzugt genannten Verbindungen der Formel I führen.

In einer erhältlichen Verbindung der Formel I, worin eine oder mehrere funktionelle Gruppen geschützt sind, können diese, z.B. geschützte Carboxyl-, Hydroxy- und/oder Aminogruppen, in an sich bekannter Weise mittels Solvolyse, insbesondere Hydrolyse, Alkoholyse oder Acidolyse, oder mittels Reduktion, insbesondere Hydrogenolyse oder chemische Reduktion, gegebenenfalls stufenweise oder gleichzeitig freigesetzt werden.

In einer verfahrensgemäss erhältlichen Verbindung der Formel I, worin $R_2$ eine geschützte Carboxylgruppe bedeutet und/oder worin der Rest $R_3$ geschütztes Carboxyl als Substituenten enthält, kann die geschützte Carboxylgruppe in an sich bekannter Weise freigesetzt werden. So kann man in 2-Stellung durch Triniederalkylsilyl substituiertes Aethoxycarbonyl z.B. durch Behandeln mit einer Carbonsäure, wie Ameisensäure oder Trifluoressigsäure, gegebenenfalls unter Zugabe einer nucleophilen Verbindung, wie Phenol oder Anisol, in freies Carboxyl überführen. Gegebenenfalls substituiertes Benzyloxycarbonyl kann z.B. mittels Hydrogenolyse, d.h. durch Behandeln mit Wasserstoff in Gegenwart eines metallischen Hydrierkatalysators, wie eines Palladiumkatalysators, gespalten werden. Ferner kann man geeignet substituiertes Benzyloxycarbonyl, wie 4-Nitrobenzyloxycarbonyl, auch mittels chemischer Reduktion, z.B. durch Behandeln mit einem Alkalimetall-, z.B. Natriumdithionit, oder mit einem reduzierenden Metall, z.B. Zinn, üblicherweise in Gegenwart eines Wasserstoff-abgebenden Mittels, das zusammen mit dem Metall nascierenden Wasserstoff zu erzeugen vermag, wie einer geeigneten Carbonsäure, z.B. einer gegebenenfalls, z.b. durch Hydroxy, substituierten Niederalkancarbonsäure, z.B. Essigsäure, oder eines Alkohols oder Thiols, wobei man vorzugsweise Wasser zugibt, in freies Carboxyl überführen. Die Abspaltung einer Allylschutzgruppe kann z.B. durch Umsetzung mit einer Verbindung des Palladiums, z.B. Tetrakis-(triphenylphosphin)-palladium, gegebenenfalls in Gegenwart von Triphenylphosphin und unter Zusatz eines Allylgruppenakzeptors, wie einer Carbonsäure, z.B. 2-Aethylhexansäure, oder eines Salzes davon oder Dimedon oder Tributylzinnhydrid, erfolgen. Durch Behandeln mit einem reduzierenden Metall oder Metallsalz, wie oben beschrieben, kann man auch 2-Halogenniederalkoxycarbonyl (gegebenenfalls nach Umwandlung einer 2-Bromniederalkoxycarbonylgruppe in eine entsprechende 2-Jodniederalkoxycarbonylgruppe) in freies Carboxyl umwandeln. Substituiertes 2-Silyläthoxycarbonyl kann auch durch Behandeln mit einem das Fluoridanion liefernden Salz der Fluorwasserstoffsäure, wie einem Alkalimetallfluorid, z.B. Natriumfluorid, in Anwesenheit eines makrocyclischen Polyäthers ("Kronenäther") oder mit einem Fluorid einer organischen quaternären Base, wie Tetraniederalkylammoniumfluorid, z.B. Tetrabutylammoniumfluorid, in freies Carboxyl überführt werden.

Andererseits können auch Verbindungen der Formel I, worin $R_2$ Carboxyl, bedeutet, in Verbindungen der Formel I überführt werden, worin $R_2$ eine unter physiologischen Bedingungen spaltbare veresterte Carboxylgruppe darstellt. So kann man die freie Carboxylgruppe z.B. durch Umsetzen mit einem zur Veresterung geeigneten Alkohol in Gegenwart eines Veresterungsmittels, wie eines Carbodiimids, z.B. Dicyclohexylcarbodiimid, oder Carbonyldiimidazol, verestern. Ester können auch durch Umsetzung eines gegebenenfalls in situ hergestellten Salzes der Säure mit einem reaktionsfähigen Ester eines Alkohols und einer starken anorganischen Säure, wie Schwefelsäure, oder einer starken organischen Sulfonsäure, wie 4-Toluolsulfonsäure, hergestellt werden.

In einer erfindungsgemäss erhältlichen Verbindung der Forml I mit einer geschützten Aminogruppe kann diese in an sich bekannter Weise, z.B. je nach Art der Schutzgruppe, vorzugsweise mittels Solvolyse oder Reduktion, in die freie Aminogruppe übergeführt werden. Beispielsweise kann 2-Halogenniederalkoxycarbonylamino (gegebenenfalls nach Umwandlung einer 2-Bromniederalkoxycarbonylaminogruppe in eine 2-Jodniederalkoxycarbonylaminogruppe) oder 4-Nitrobenzyloxycarbonylamino durch Behandeln mit einem geeigneten chemischen Reduktionsmittel, wie Zink in schwach saurem Milieu, wie in Gegenwart einer geeigneten Carbonsäure, z.B. wässriger Essigsäure, oder in einem sauren Phosphat-Puffer, z.B. Kaliumdihydrogenphosphat-Puffer, oder katalytisch mit Wasserstoff in Gegenwart eines Palladiumkatalysators gespalten werden. 4-Nitrobenzyloxycarbonylamino kann auch durch Behandeln mit einem Alkalimetall-, z.B. Natriumdithionit gespalten werden. Gegebenenfalls substituiertes Benzyloxycarbonylamino kann z.B. mittels Hydrogenolyse, d.h. durch Behandeln mit Wasserstoff in Gegenwart eines geeigneten Hydrierkatalysators, wie eines Palladiumkatalysators, und Allyloxycarbonylamino durch Umsetzen mit einer Verbindung des Palladiums, z.B. Tetrakis(triphenylphosphin)palladium, gegebenenfalls in Gegenwart von Triphenylphosphin und in Gegenwart eines Allylgruppenakzeptors, wie einer Carbonsäure, z.B. 2-Aethylhexansäure, oder eines Salzes davon oder Dimedon oder Tributylzinnhydrid, gespalten werden. Eine durch 2-substituiertes Silyläthoxycarbonyl geschützte Aminogruppe kann durch Behandeln mit einem Fluoridanion liefernden Salz der Fluorwasserstoffsäure, wie einem Alkalimetallfluorid, z.B. Natriumfluorid, in Anwesenheit eines makrocyclischen Polyäthers ("Kronenäther") oder mit einem Fluorid einer organischen quaternären Base, wie Tetraniederalkylammoniumfluorid, z.B. Tetraäthylammoniumfluorid, in die freie Aminogruppe übergeführt werden. Eine in Form einer Azidogruppe geschützte Aminogruppe wird z.B. durch Reduktion in freies Amino überführt, beispielsweise durch katalytische Hydrierung mit Wasserstoff in Gegenwart eines Hydrierkatalysators, wie Platinoxid oder Palladium, oder durch Behandeln mit Zink in Gegenwart einer Säure, wie Essigsäure.

In verfahrensgemäss erhältlichen Verbindungen der Formel I, worin der Rest $R_1$ und/oder der Rest $R_3$ geschütztes Hydroxyl als Substituenten enthält, kann die geschützte Hydroxygruppe in an sich bekannter Weise in die freie Hydroxygruppe überführt werden. Beispielsweise wird eine durch eine geeignete Acylgruppe oder eine organische Silylgruppe geschützte Hydroxygruppe wie eine entsprechend geschützte Aminogruppe freigesetzt (s.o.); eine Triniederalkylsilylgruppe kann z.B. auch mit Tetrabutylammoniumfluorid und Essigsäure abgespalten werden (unter diesen Bedingungen werden durch trisubstituiertes Silyläthoxy geschützte Carboxygruppen nicht gespalten).

In erhältlichen Verbindungen der Formel I kann man weiterhin einen Rest $R_3$ in einen anderen Rest $R_3$ überführen.

So kann man in Verbindungen der Formel I, worin $R_3$ für die Gruppe $-Y-N(R_4,R_5)$ steht, einen Rest $R_4$ und/oder $R_5$ und/oder Y in einen anderen Rest $R_4$ und/oder $R_5$ und/oder Y überführen.

In Verbindungen der Formel I, worin der Rest Y durch eine Carboxylgruppe substituiert ist, kann diese Carboxylgruppe nach an sich bekannten Verfahren in eine funktionell abgewandelte Carboxylgruppe, wie z.B. in eine veresterte Carboxylgruppe oder in gegebenenfalls substituiertes Carbamoyl, überführt werden. Beispielsweise erhält man durch Umsetzen einer Verbindung der Formel I, worin Y eine Gruppe der Formel $-CR_6R_7-$ ist und $R_6$ ein durch Carboxy substituierter organischer Rest ist, mit einem Niederalkanol eine Verbindung der Formel I, worin Y eine Gruppe der Formel $-CR_6R_7-$ ist und $R_6$ ein durch Niederalkoxycarbonyl substituierter organischer Rest ist, wobei man vorzugsweise in Gegenwart eines geeigneten Kondensationsmittels, z.B. eines Carbodiimids, arbeitet oder das entstehende Wasser z.B. durch azeotrope Destillation entfernt. Andererseits kann man Carboxylgruppen im Rest Y auch in reaktionsfähige funktionelle Derivate, wie gemischte Anhydride, z.B. Säurehalogenide, oder aktivierte Ester, überführen und diese durch Umsetzen mit einem Niederalkanol, Ammoniak oder einem primären oder sekundären Amin, z.B. einem Niederalkyl- oder Diniederalkylamin, in entsprechend veresterte oder amidierte Carboxylgruppen umwandeln, wobei man bei Verwendung von gemischten Anhydriden vorzugsweise in Gegenwart eines säurebindenden Mittels, wie eines aromatischen oder tertiären Amins oder eines Alkalimetall-oder Erdalkalimetallcarbonats, arbeitet.

Weist eine (Hetero)aryl-Gruppe im Rest Y eine Hydroxygruppe auf, so lässt sich diese auf üblichem Wege veräthern. Die Umsetzung zu den entsprechenden Niederalkyl-(hetero)aryl-äthern erfolgt beispielsweise in Gegenwart von Basen, wie Alkalimetallhydroxiden oder -carbonaten, z.B. Natriumhydroxid oder Kaliumcarbonat, mit Hilfe von Diniederalkylsulfaten oder Niederalkylhalogeniden. Weiterhin lässt sich Hydroxy in verestertes Hydroxy, z.B. Niederalkanoyloxy, umwandeln, beispielsweise durch Umsetzung mit dem reaktionsfähigen Derivat einer entsprechenden Niederalkancarbonsäure, z.B. Essigsäure, wie einem Anhydrid davon, z.B. dem symmetrischen Anhydrid davon oder einem gemischten Anhydrid mit einer Halogenwasserstoffsäure, erforderlichenfalls in Gegenwart eines basischen Kondensationsmittels, wie eines Alkalimetallhydroxids oder -carbonats, oder eine Stickstoffbase, z.B. Pyridin. Die Umwandlung von Niederalkanoyloxy zu Hydroxy erfolgt beispielsweise durch Alkoholyse oder, vorzugsweise, Hydrolyse, z.B. durch basenkatalysierte Hydrolyse, z.B. in Gegenwart von Natriumhydroxid.

In Verbindungen der Formel I mit einer freien Aminogruppe kann die Aminogruppe in eine substituierte Aminogruppe, z.B. eine Niederalkylamino-, Diniederalkylamino-, Niederalkylenamino-oder Niederalkanoylaminogruppe, überführt werden. Die Ueberführung in eine Niederalkylamino- oder Diniederalkylaminogruppe erfolgt beispielsweise durch Reaktion mit einem reaktionsfähigen veresterten Niederalkanol, beispielsweise einem Niederalkylhalogenid oder -sulfonat, in Gegenwart eines basischen Kondensationsmittels, wie eines Hydroxids oder Carbonats eines Alkali-oder Erdalkalimetalls oder einer heteroaromatischen Stickstoffbase, z.B. Pyridin. In analoger Weise kann Amino durch Behandeln mit einem Niederalkylendihalogenid oder -disulfonat in Niederalkylenamino und durch Behandeln mit dem reaktionsfähigen funktionellen Derivat einer Niederalkancarbonsäure, z.B. dem entsprechenden Carbonsäurehalogenid, in Niederalkanoylamino umgewandelt werden. Verbin dungen der Formel I mit einem substituierbaren Ringstickstoffatom im Rest $R_3$ können auf die gleiche Weise in Verbindungen der Formel I überführt werden, welche im Rest $R_3$ ein durch einen gegebenenfalls substituierten Niederalkylrest substituiertes Ringstickstoffatom enthalten. Weiterhin können Verbindungen der Formel I, worin $R_5$ Wasserstoff ist, durch Umsetzen mit einem den Acylrest einer Carbonsäure, eines Halbesters der Kohlensäure, einer gegebenenfalls substituierten Carbaminsäure, Sulfonsäure oder Amidosulfonsäure, z.B. einem Säurehalogenid einer solchen Säure, z.B. dem Chlorid oder Bromid davon, einführenden Acylierungsmittel vorzugsweise in Gegenwart eines säurebindenden Mittels, wie eines der oben genannten, in Verbindungen der Formel I überführt werden, worin $R_5$ Acyl ist.

Salze von Verbindungen der Formel I mit salzbildenden Gruppen können in an sich bekannter Weise hergestellt werden. So kann man Salze von Verbindungen der Formel I mit einer freien Carboxyl- oder Sulfogruppe z.B. durch Behandeln mit Metallverbindungen, wie Alkalimetallsalzen von geeigneten organischen Carbonsäure, z.B. dem Natriumsalz der α-Aethylcapronsäure, oder mit anorganischen Alkali- oder Erdalkalimetallsalzen, z.B. Natriumhydrogencarbonat, oder mit Ammoniak oder mit einem geeigneten organischen Amin bilden, wobei man vorzugsweise stöchiometrische Mengen oder nur einen kleinen Ueberschuss des salzbildenden Mittels verwendet. Säureadditionssalze von Verbindungen der Formel I erhält man in üblicher Weise, z.B. durch Behandeln mit einer geeigneten Säure oder einem geeigneten Anionenaustauscherreagenz. Innere Salze von Verbindungen der Formel I können z.B. durch Neutralisieren von Salzen, wie Säureadditionssalzen, auf den isoelektrischen Punkt, z.B. mit schwachen Basen, oder durch Behandeln mit Ionenaustauschern gebildet werden.

Salze können in üblicher Weise in die freien Verbindungen überführt werden, Metall- und Ammoniumsalze

z.B. durch Behandeln mit geeigneten Säuren und Säureadditionssalze z.B. durch Behandeln mit einem geeigneten basischen Mittel.

Erhaltene Gemische von isomeren Verbindungen können nach an sich bekannten Methoden in die einzelnen Isomere aufgetrennt werden. Beispielsweise kann man ein erhaltenes Racemat mit einem optisch aktiven Hilfsstoff reagieren lasen, dabei entstandene Gemisch zweier diastereometer Verbindungen mit Hilfe von geeigneten physikalisch-chemischen Methoden (z.B. fraktioniertes Kristallisieren, Adsorptionschromatographie) trennen und die einzelnen diastereomeren Verbindungen dann in die optisch aktiven Verbindungen spalten.

Bei allen nachträglichen Umwandlungen erhaltener Verbindungen der Formel I werden solche Reaktionen bevorzugt, die unter gemässigt alkalischen oder insbesondere neutralen Bedingungen erfolgen.

Das Verfahren umfasst auch diejenigen Ausführungsformen, wonach als Zwischenprodukte anfallende Verbindungen als Ausgangsstoffe verwendet und die restlichen Verfahrensschritte mit diesen durchgeführt werden, oder das Verfahren auf irgendeiner Stufe abgebrochen wird. Ferner können Ausgangsstoffe in Form von Derivaten verwendet oder in situ, gegebenenfalls unter den Reaktionsbedingungen, hergestellt werden.

Die Ausgangsverbindungen der Formeln II und III können, wie in dem folgenden Reaktionsschema I angegeben, hergestellt werden:

Reaktionsschema I

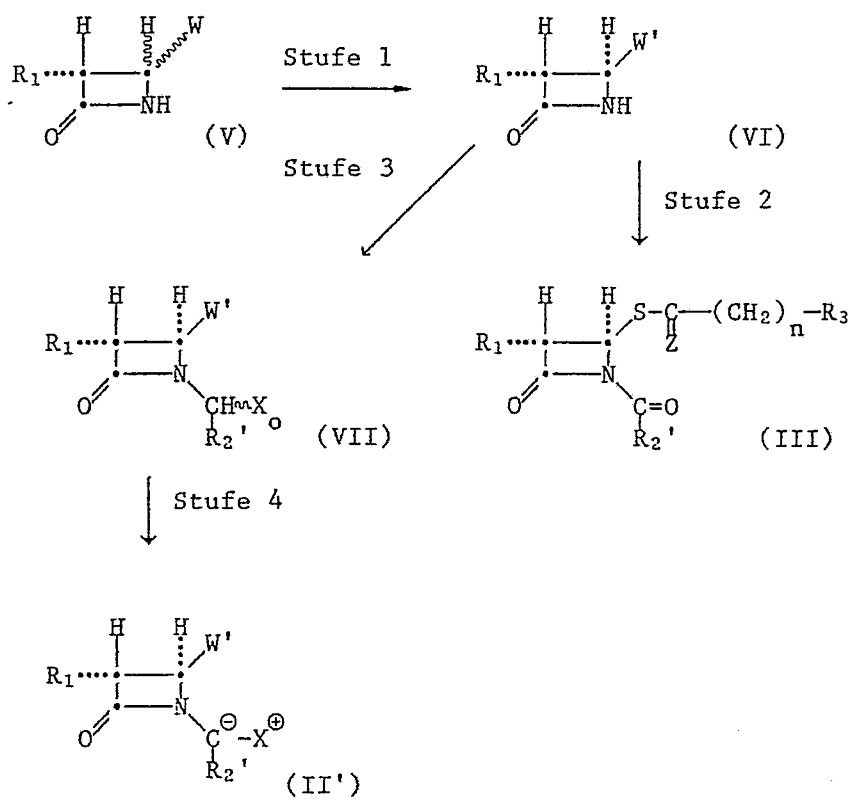


In den Verbindungen der Formeln II', VI und VII steht W' für $-S-C(=Z)-(CH_2)_n-R_3$ oder für Triphenylmethylthio oder Niederalkanoylthio.

Stufe 1

Geeignete Ausgangsverbindungen der Formel V, worin W ein durch nucleophile Reaktion leicht austauschbarer Rest, z.B. Niederalkanoyloxy, wie Acetyloxy, oder Sulfonyloxy $R-SO_2-$, worin R z.B. gegebenenfalls durch Hydroxy substituierten Niederalkyl, wie Methyl, tert.Butyl oder 2-Hydroxyäthyl, ist, sind beispielsweise aus der veröffentlichten Europäischen Patentanmeldung Nr. 82113, der Deutschen Offenlegungsschrift Nr. 3 224 055 und der Deutschen Offenlegungsschrift Nr. 3 013 997 bekannt oder können in dazu analoger Weise hergestellt werden.

Eine den Rest $-S-C(=Z)-(CH_2)_n-R_3$ einführende Verbindung ist beispielsweise eine Säure der Formel $R_3-(CH_2)_n-C(=Z)-SH$ oder insbesondere ein Salz, z.B. ein Alkalimetallsalz, wie das Natrium-oder Kaliumsalz,

davon. Die Substitution kann in einem organischen Lösungsmittel, wie in einem Niederalkanol, einem Niederalkancarbonsäureamid, einem cyclischen Aether, oder in einem ähnlichen inerten Lösungsmittel bei Raumtemperatur oder bei etwas erhöhter oder erniedrigter Temperatur, z.B. bei etwa 0° bis etwa 40° C, durchgeführt werden. Die Einführung eines Triphenylmethylthio-oder Niederalkanoylthio-Restes W' erfolgt in analoger Weise durch Umsetzen mit einem Alkalimetallsalz, z.B. dem Natriumsalz, einer Niederalkanthiocarbonsäure, z.B. Thioessigsäure, oder des Triphenylmethylmercaptans.

Stufe 2

Eine Ausgangsverbindung der Formel (III) wird erhalten, indem man ein Azetidinon der Formel (VI), in der W' für den Rest -S-C(=Z)-$(CH_2)_n$-$R_3$ steht, mit einer Säure der Formel $R_2'$-COOH oder insbesondere einem reaktionsfähigen Derivat, wie einem Ester oder Säurehalogenid, z.B. dem Säurechlorid, davon bei einer Temperatur von -50° bis 80° C, bevorzugt bei -20° bis 0° C, in einem inerten Lösungsmittel, wie einem der bei der Umsetzung von Verbindungen der Formel (III) zu Verbindungen der Formel I genannten, behandelt. Bei Verwendung eines Säurehalogenids arbeitet man vorzugsweise in Gegenwart eines säurebindenden Mittels, wie eines tertiären aliphatischen Amins, eines aromatischen Amins, oder insbesondere eines Alkalimetall- oder Erdalkalimetallcarbonats oder -hydrogencarbonats.

Verbindungen der Formel (VI), worin W' für Triphenylmethylthio oder Niederalkanoylthio steht, können in die Ausgangsverbindungen der Formel VI, worin W' für den Rest -S-C(=Z)-$(CH_2)_n$-$R_3$ steht, überführt werden, indem man sie in Gegenwart einer Base, z.B. Pyridin oder Tri-n-butylamin, in einem geeigneten Lösungsmittel, z.B. Diäthyläther oder Methanol, mit einem Salz der Formel MA, worin M für ein Uebergangsmetallkation, insbesondere für das Silberkation, steht, und A ein gebräuchliches Anion darstellt, welches die Löslichkeit des Salzes M in dem gewählten Lösungsmittel begünstigt, z.B. das Nitrat-, Acetat-oder Fluorid-Anion, umsetzt, und das entstandene Salz der Formel

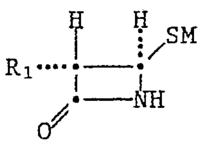
(VIII),

mit einem den Rest $R_3$-$(CH_2)_n$-C(=Z)- einführenden Acylierungsmittel, z.B. mit der Säure $R_3$-$(CH_2)_n$-C(=Z)-OH oder einem reaktionsfähigen funktionellen Derivat, wie einem Säurehalogenid, z.B. dem Chlorid oder Bromid, Azid oder Anydrid davon, behandelt. Die Acylierung erfolgt, wenn ein reaktionsfähiges funktionelles Derivat der Säure der Formel $R_3$-$(CH_2)_n$-C(=Z)-OH, z.B. das Säurechlorid, eingesetzt wird, in einem inerten Lösungsmittel, wie einem chlorierten Kohlenwasserstoff, oder einem Aether, bei Raumtemperatur oder unter Erhitzen oder Kühlen, z.B. in einem Temperaturbereich von ca. -50° bis ca. +60° C, insbesondere bei ca. -30° bis ca. +20° C.

Stufe 3

Verbindungen der Formel VII, worin $X_0$ für eine reaktionsfähige veresterte Hydroxygruppe, insbesondere für Halogen, z.B. Chlor oder Brom, steht, werden hergestellt, indem man eine Verbindung der Formel VI mit einer Glyoxylsäure-Verbindung der Formel $R_2'$-CHO oder einem geeigneten Derivat davon, wie einem Hydrat, Hemihydrat oder Halbacetal, z.B. einem Halbacetal mit einem Niederalkanol, z.B. Methanol oder Aethanol, umsetzt, und in einer erhaltenen Verbindung der Formel XII, worin $X_0$ für Hydroxy steht, die Hydroxygruppe in eine reaktionsfähige veresterte Hydroxygruppe umwandelt. Die Verbindungen der Formel VII werden üblicherweise als Gemisch der beiden Isomere [bezüglich der Gruppierung -CH($R'_2$) ~ $X_0$] erhalten.

Die Anlagerung der Glyoxylsäureester-Verbindung an das Stickstoffatom des Lactamrings in der Verbindung der Formel VI findet bei Raumtemperatur oder, falls notwendig, unter Erwärmen, statt. Bei Verwendung des Hydrats der Glyoxylsäure-Verbindung wird Wasser gebildet, das, wenn notwendig, durch Destillation, z.B. azeotrop, oder durch Verwendung eines geeigneten Dehydrationsmittels entfernt wird. Vorzugsweise arbeitet man in Gegenwart eines geeigneten inerten Lösungsmittels oder Lösungsmittelgemisches.

Die Ueberführung einer Hydroxygruppe $X_0$ in eine reaktionsfähige veresterte Hydroxygruppe $X_0$ in einer Verbindung der Formel VII wird durch Behandeln mit einem geeigneten Veresterungsmittel durchgeführt, z.B. mit einem Thionylhalogenid, z.B. -chlorid, vorzugsweise in Gegenwart eines basischen, in erster Linie organischen basischen Mittels, wie eines aliphatischen tertiären Amins, oder einer heterocyclischen Base vom Pyridintyp. Vorzugsweise arbeitet man in Gegenwart eines geeigneten Lösungsmittels, z.B. Dioxan oder Tetrahydrofuran, oder eines Lösungsmittelgemisches, wenn notwendig unter Kühlen, z.B. bei etwa -30° bis etwa 30° C.

Stufe 4

Das Ausgangsmaterial der Formel II wird erhalten, indem man eine Verbindung der Formel VII mit einer geeigneten Phosphin-Verbindung, wie einem Triniederalkylphosphin, z.B. Tri-n-butyl-phosphin, oder einem Triaryl-phosphin, z.B. Triphenylphosphin, oder mit einer geeigneten Phosphit-Verbindung, wie einem Triniederalkylphosphit, z.B. Triäthylphosphit, oder einem Alkalimetalldiniederalkylphosphit, z.B. -diäthylphosphit, behandelt, und eine gegebenenfalls erhältlichen Verbindung der Formel II' worin W' für Triphenylmethylthio oder Niederalkanoylthio steht, in eine Verbindung der Formel II überführt, worin W' für den Rest -S-C(=Z)-$(CH_2)_n$-$R_3$ steht.

Die Umsetzung mit der Phosphin- bzw. Phosphit-Verbindung wird vorzugsweise in einem geeigneten inerten Lösungsmittel, wie einem Kohlenwasserstoff, oder einem Aether oder in einem Lösungsmittelgemisch vorgenommen. Je nach Reaktionsfähigkeit arbeitet man unter Kühlen oder bei erhöhter Temperatur, etwa zwischen -10° und +100°C, bevorzugt bei etwa 20° bis 80°C. Ueblicherweise arbeitet man in Gegenwart eines basischen Mittels, wie einer organischen Base, z.B. eines Amins, oder "Polystyol-Hünigbase", oder einer anorganischen Base, z.B. eines Alkalimetallcarbonats, wobei die primär entstandene Phosphoniumverbindung der Formel

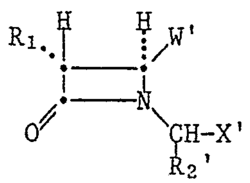

(IIa),

worin X' eine Phosphonogruppe oder eine Phosphoniogruppe zusammen mit einem Anion, je nach Bedeutung des Restes $X_0$ (vgl. Formel VII) z.B. Chlorid, bedeutet, in das Ylid-Ausgangsmaterial der Formel II umgewandelt wird.

Die Einführung des Restes -S-C(=Z)-$CH_2)_n$-$R_3$ in Verbindungen der Formel II', worin W' für Niederalkanoylthio oder Triphenylmethylthio steht, kann in analoger Weise erfolgen, wie unter Stufe 2 beschrieben.

Man kann das in Reaktionsschema I beschriebene Verfahren zur Herstellung der Verbindungen der Formeln (II), (III), (VI) und (VII), sowie das angegebene Verfahren zur Herstellung der Endprodukte der Formel (I) auch mit optisch inaktiven Verbindungen durchführen und auf einer beliebigen Verfahrensstufe aus einem erhaltenen Diastereomerengemisch oder Racemat in bekannter Weise die optisch aktiven Verbindungen gemäss vorliegender Erfindung isolieren.

Die Ausgangsverbindungen der Formel IA sind aus dem Japanischen Patent Nr. 56/166194 [Chemical Abstracts 96, 142565u (1982)] bekannt oder können in analoger Weise zu den dort beschriebenen Verfahren hergestellt werden.

Die Ausgangsverbindungen der Formel IV sind bekannt oder können in analoger Weise zu den in der Literatur beschriebenen Verfahren hergestellt werden.

Die Erfindung betrifft ebenfalls die neuen Ausgangsverbindungen sowie verfahrensgemäss erhältliche neue Zwischenprodukte, wie diejenigen der Formeln II, III, ferner VI und VII [W' steht für den Rest -S-C(=Z)-$(CH_2)_n$-$R_3$] sowie die angegebenen Verfahren zu ihrer Herstellung.

Vorzugsweise werden solche Ausgangsverbindungen verwendet und die Reaktionsbedingungen so gewählt, dass man zu den vorstehend als besonders bevorzugt aufgeführten Verbindungen der Formel I gelangt.

Die pharmakologisch verwendbaren Verbindungen der vorliegenden Erfindung können z.B. zur Herstellung von pharmazeutischen Präparaten verwendet werden, welche eine therapeutisch wirksame Menge der Aktivsubstanz zusammen oder im Gemisch mit anorganischen oder organischen, festen oder flüssigen, pharmazeutisch annehmbaren Trägerstoffen enthalten, die sich zur oralen oder zur parenteralen, d.h. z.B. intramuskulären, intravenösen, subcutanen oder intraperitonealen, Verabreichung eignen.

Zur oralen Verabreichung verwendet man Tabletten oder Gelatinekapseln, welche den Wirkstoff zusammen mit Verdünnungsmitteln, z.B. Lactose, Dextrose, Sucrose, Mannitol, Sorbitol, Cellulose und/oder Glycin, und Schmiermitteln, z.B. Kieselerde, Talk, Stearinsäure oder Salze davon, wie Magnesium- oder Calciumstearat, und/oder Polyäthylenglykol, aufweisen. Tabletten enthalten ebenfalls Bindemittel, z.B. Magnesiumaluminiumsilikat, Stärken, wie Mais-, Weizen-, Reis-oder Pfeilwurzstärke, Natriumcarboxymethyl-stärke, Gelatine, Tragacent, Methylcellulose, Natriumcarboxymethylcellulose und/oder Polyvinylpyrrolidon, und, wenn erwünscht, Sprengmittel, z.B. Stärken, Agar, Alginsäure oder Salze davon, wie Natriumalginat, und/oder Brausemischungen oder Adsorptionsmittel, Farbstoffe, Geschmackstoffe oder Süssmittel.

Zur parenteralen Verabreichung eignen sich in erster Linie Infusionslösungen, vorzugsweise isotonische wässrige Lösungen oder Suspensionen, wobei diese z.B. aus lyophilisierten Präparaten, welche die Wirksubstanz allein oder zusammen mit einem Trägermaterial, z.B. Mannit enthalten, vor Gebrauch hergestellt werden können. Solche Präparate können sterilisiert sein und/oder Hilfsstoffe, z.B. Konservier-, Stabilisier-, Netz- und/oder Emulgiermittel, Löslichkeitsvermittler, Salze zur Regulierung des osmotischen Druckes

und/oder Puffer enthalten.

Die vorliegenden pharmazeutischen Präparate, die, wenn erwünscht, weitere pharmakologisch wertvolle Stoffe enthalten können, werden in an sich bekannter Weise, z.B. mittels konventioneller Mischungs-, Lösungs-oder Lyophilisierungsverfahren, hergestellt und enthalten von etwa 0,1 % bis 100 %, insbesondere von etwa 1 % bis etwa 50 %, Lyophilisate bis zu 100 % des Aktivstoffs.

Je nach Art der Infektion und Zustand des infizierten Organismus verwendet man tägliche Dosen (oral oder parenteral) von etwa 100 mg bis etwa 1 g zur Behandlung von Warmblütern (Menschen und Tiere) von etwa 70 kg Gewicht.

Die folgenden Beispiele dienen zur Illustration der Erfindung. Temperaturen werden in Celsiusgraden angegeben.

Beispiel 1:
(5R,6S)-2-[2-(tert.-Butyldimethylsiloxy)-äthyl]-6-[(1R)-1-allyloxycarbonyloxyäthyl]-2-penem-3-carbonsäureallylester

Eine Lösung von 9,46 g 2-[(3S,4R)-3-[(1R)-1-Allyloxycarbonyloxyäthyl]-4-[3-(tert.-butyldimethylsilyloxy)-propionylthio]-2-oxoazetidin-1-yl]-2-triphenylphosphoranylidenessigsäureallylester in 2 l Toluol wird unter Argonatomsphäre 5 ½ Stunden bei einer Badtemperatur von 110° gerührt. Dann wird das Lösungsmittel abgedampft und das Rohprodukt durch Chromatographie an Silicagel gereinigt (Laufmittel Toluol/Aethylacetat 9:1).

$R_f$ (Toluol/Aethylacetat 1:1) = 0,7.

IR ($CH_2Cl_2$) 1790; 1745; 1705 $cm^{-1}$.

Das Ausgangsmaterial kann wie folgt hergestellt werden:

a. 3-Hydroxy-propionsäure

In 800 ml Wasser werden 54 ml (0,8 mol) 3-Hydroxy-propionitril und 80 g Natriumhydroxid 16 Stunden bei 80° gerührt. Anschliessend wird im Vakuum auf ein Volumen von ca 300 ml konzentriert und im Eisbad innert 1 Stunde mit ca. 160 ml Salzsäure neutralisiert (pH 3). Das viskose Reaktionsgemisch wird mit 400 ml Aethylacetat versetzt, filtriert und erschöpfend mit total 5 l Aethylacetat extrahiert. Die vereinigten organischen Phasen werden über Natriumsulfat getrocknet und das Lösungsmittel bei max. 50° Badtemperatur abdestilliert. Die erhaltene rohr 3-Hydroxypropionsäure kann direkt weiter werwendet werden.

b. 3-(tert.-Butyldimethylsilyloxy)-propionsäure

10 g β-Hydroxypropionsäure (Rohprodukt) werden in 50 ml Dimethylformamid gelöst und bei 0° mit 19,3 g Imidazol und 18,8 g tert.-Butyldimethylchlorsilan versetzt. Nach weiteren 30 Minuten bei 0° wird 48 Stunden bei 22° gerührt. Zur Aufarbeitung wird das Reaktionsgemisch auf eine Mischung bestehend aus 500 ml Aethylacetat, 100 ml 2N wässriger Salzsäure und 100 g Eis gegossen, die wässrige Phase wird abgetrennt und noch zweimal mit Aethylacetat nachge waschen. Die vereinigten organischen Phasen werden mehrmals mit Wasser neutral gewaschen, über $Na_2SO_4$ getrocknet und das Lösungmittel abgedampft. Das Rohprodukt wird am Hochvakuum über eine kurze Vigreux-Kolonne destilliert, Kp. 84°/0,02 Torr.

Zur Abtrennung von als Verunreinigung enthaltener Bis-silylierter 3-Hydroxypropionsäure wird 14,6 g des obigen Destillats in 200 ml Aethylacetat aufgenommen und mit 60 ml 1N wässriger Natronlauge bei 0° versetzt. Die wässrige Phase wird abgetrennt, einmal mit wenig Aethylacetat nachgewaschen und mit 60 ml 1N eiskalter wässriger Salzsäure angesäuert. Die freie Säure wird mit Aethylacetat extrahiert, die vereinigten organischen Phasen über $Na_2SO_4$ getrocknet und das Lösungsmittel abgedampft. Nach Kugelrohrdestillation bei einer Ofentemp. von 100-110° und 0,01 Torr wird die reine Titelverbindung isoliert. $^1H$-NMR (60 MHz,$CDCl_3$): δ=9,3(s,br); 3,86(t,J=9 Hz); 2,52(t,J=9 Hz); 0,83(s); 0,00(s) ppm.

c. 3-(tert.-Butyldimethylsiloxyloxy)-propionylchlorid

Eine Lösung von 8,59 g 3-(tert.-Butyldimethylsilyloxy)-propionsäure in 213 ml Methylenchlorid wird bei Raumtemperatur und unter Stickstoffatmosphäre mit 6,41 ml 1-Chlor-1-dimethylamino-2-methyl-prop-1-en versetzt. Nach 15 Minuten wird mit Hilfe eines IR-Spektrums der Reaktionslösung festgestellt, dass die Umsetzung vollständig ist (Säurechlorid-Bande bei 1793 $cm^{-1}$). Diese Lösung wird direkt in die nächste Stufe eingesetzt.

d. 2-[(3S,4R)-3-[(1R)-1-Allyloxycarbonyloxyäthyl]-4-[3-(tert.-butyldimethylsilyloxy)-propionylthio]-2-oxo-azetidin-1-yl]-2-triphenylphosphoranylidenessigsäureallylester

18,59 g Silbersalz des 2-[(3S,4R)-4-[(1R)-1-Allyloxycarbonyloxyäthyl]-4-mercapto-2-oxo-azetidin-1-yl]-2-triphenylphosphoranylidenessigsäureallylesters werden in 320 ml Methylendichlorid gelöst, bei 0° mit 4,35 ml Pyridin, 535 mg 4-Dimethylaminopyridin und anschliessend tropfenweise mit einer in situ hergestellten Lösung von 9,38 g 3-(tert.-Butyldimethylsilyloxy)-propionylchlorid in 213 ml Methylenchlorid versetzt. Nach 45 Minuten

Rühren bei 0° wird der Feststoff über Hyflo abfiltriert und das Filtrat wird mit 5 %iger wässriger Natriumcarbonat Lösung und anschliessend zweimal mit Sole gewaschen. Nach Trocknen über $Na_2SO_4$ wird das Lösungsmittel abgedampft. Der Rückstand wird durch Chromatographie an Silicagel (Laufmittel Toluol/Aethylacetat 4:1) gereinigt.
$R_f$ (Toluol/Aethylacetat 1:1) = 0,4.
IR ($CH_2Cl_2$): 1755, 1750, 1620 $cm^{-1}$.

Beispiel 2: (5R,6S)-2-(2-Hydroxyäthyl)-6-[(1R)-1-allyloxycarbonyloxyäthyl]-2-penem-3-carbonsäureallylester

In 66,6 ml Tetrahydrofuran werden 3,55 g (5R,6S)-2-(2-tert.-Butyldimethylsilyloxyäthyl)-6-[(1R)-1-allyloxycarbonyloxyäthyl]-2-penem-3-carbonsäureallylester gelöst und bei -60° mit 3,1 ml Eisessig unter Stickstoffatmosphäre versetzt. Anschliessend werden 15,9 ml einer 1 M Tetra-n-butyl-ammoniumfluoridlösung in Tetrahydrofuran verdünnt mit 88,4 ml Tetrahydrofuran langsam zugetropft. Anschliessend lässt man auf Raumtemperatur aufwärmen. Nach 3 $\frac{1}{2}$ Stunden bei Raumtemperatur wird am Vakuum das Lösungsmittel abdestilliert und der Rückstand in 100 ml Aethylacetat aufgenommen. Die organische Phase wird zweimal mit eiskalter, wässriger 10 %iger Natriumhydrogencarbonatlösung und einmal mit Sole gewaschen. Nach Trocknen über $Na_2SO_4$ wird das Lösungsmittel abgedampft. Der Rückstand wird durch Chromatographie an Silicagel (Laufmittel Toluol/Aethylacetat 4:1→3:2) gereinigt.
DC (Toluol/Aethylacetat 1:1) $R_f$ = 0,3.
IR ($CH_2Cl_2$): 1790, 1745; 1710 $cm^{-1}$.
UV (Aethanol): $\lambda_{max}$ = 315 nm.

Beispiel 3:
(5R,6S)-2-[2-(N-Allyloxycarbonylglycyloxy)-äthyl]-6-[(1R)-1-allyloxycarbonyloxyäthyl]-2-penem-3-carbonsäureallylester

Eine Lösung von 95,5 mg (5R,6S)-2-(2-Hydroxyäthyl)-6-[(1R)-1-allyloxycarbonyloxyäthyl]-2-penem-3-carbonsäureallylester in 2,5 ml abs. Methylenchlorid wird bei Raumtemperatur mit 43,7 mg N-Allyloxycarbonylglycin und 2 mg 4-Dimethylaminopyridin versetzt. Anschliessend werden 56,6 mg Dicyclohexylcarbodiimid bei Raumtemperatur zugegeben, und die erhaltene Suspension wird 2 $\frac{1}{2}$ Stunden bei gleicher Temperatur weiter gerührt. Der Feststoff wird danach abfiltriert und mit wenig Methylenchlorid nachgewaschen. Das Filtrat wird mit wässrigem Natriumhydrogencarbonat und Sole je einmal gewaschen. Nach Trocknen der organischen Phase über Natriumsulfat wird das Lösungsmittel abdestilliert. Die Titelsubstanz wird nach Chromatographie an 10 g Kieselgel mit Toluol/Aethylacetat 4:1 erhalten.
DC (Toluol/Aethylacetat 1:1) $R_f$ = 0,5.
IR ($CH_2Cl_2$) 3440; 1790; 1745; 1725 $cm^{-1}$.
UV (Aethanol) $\lambda_{max}$ = 316 nm.

Beispiel 4: (5R,6S)-2-(2-Glycyloxyäthyl)-6-[(1R)-1-hydroxyäthyl]-2-penem-3-carbonsäure

Eine Lösung von 306 mg (5R,6S)-2-[2-(N-Allyloxycarbonylglycyloxy)-äthyl]-6-[(1R)-1-allyloxycarbonyloxyäthyl]-2-penem-3-carbonsäureallylester in 5,3 ml abs. THF wird unter Stickstoffatmosphäre mit 178,9 mg Dimedon und 40 mg Tetrakis-triphenylphosphin-palladium versetzt. Man lässt 35 Minuten bei Raumtemperatur rühren. Anschliessend wird die ausgefallene, rohe Titelverbindung abfiltriert, mit 1 ml Tetrahydrofuran und anschliessend 10 ml Hexan gewaschen und am Vakuum getrocknet. Reinigung durch Chromatographie an Opti $UPC_{12}$ (Laufmittel Wasser) ergibt die reine Titelsubstanz.
DC (Opti $UPC_{12}$; Wasser) $R_f$ = 0,4
IR (DMSO-$d_6$): 3430; 1777; 1753; 1615, 1585 $cm^{-1}$.
UV (Wasser) $\lambda_{max}$ = 304 nm.

Beispiel 5:
(5R,6S)-2-{2-[4-(N-Allyloxycarbonylamino)-butyryloxy]-äthyl}-6-[(1R)-1-allyloxycarbonyloxyäthyl]-2-penem-3-carbonsäureallylester

Analog Beispiel 3 werden 191 mg (5R,6S)-2-(2-Hydroxyäthyl)-6-[(1R)-1-allyloxycarbonyloxyäthyl]-2-penem-3-carbonsäureallylester mit 102,9 mg 4-(N-Allyloxycarbonylamino)-buttersäure zur Titelverbindung umgesetzt.
IR ($CH_2Cl_2$): 3440; 1790; 1735; 1720; 1245 $cm^{-1}$.
UV (Aethanol) $\lambda_{max}$ = 314 nm.

Beispiel 6: (5R,6S)-2-[2-(4-Amino-butyryloxy)-äthyl]-6-[(1R)-1-hydroxyäthyl]-2-penem-3-carbonsäure

In 11,5 ml THF werden 205 mg (5R,6S)-2-{2-[4-(N-Allyloxycarbonylamino)-butyryloxy]-äthyl}-6-[(1R)-1-allyloxycarbonyloxyäthyl]-2-penem-3-carbonsäure gelöst und bei Raumtemperatur unter Stickstoffatmosphäre nacheinander mit 26,4 mg Tetrakis-triphenylphosphinpalladium und 0,409 ml Tri-n-butyl-zinnhydrid versetzt. Nach 30 Minuten werden 95,7 µl Essigsäure zugegeben und 15 Minuten nachgerührt. Dann wird am Vakuum

das THF abdesilliert, der Rückstand in Aethylacetat und wenig Wasser aufgenommen und mit wässriger Natriumhydrogencarbonatlösung auf einen pH von 6 bis 7 gestellt. Die wässrige Phase wird abgetrennt, mit wenig Aethylacetat nachgewaschen und am Vakuum bei Raumtemperatur auf ein Volumen von ca. 1 ml konzentriert. Aus dieser wässrigen Lösung wird durch Chromatographie an Opti $UPC_{12}$ (Laufmittel Wasser) die reine Titelverbindung erhalten.
IR ($DMSO-d_6$): 3415; 3300; 1768; 1734; 1612; 1583 $cm^{-1}$.
UV (Wasser) $\lambda_{max}$ = 303 nm.

Beispiel 7:
(5R,6S)-2-[2-(β-N-Allyloxycarbonylalanyloxy)-äthyl]-6-[(1R)-1-allyloxycarbonyloxyäthyl]-2-penem-3-carbonsäureallylester

Analog Beispiel 3 werden 191 mg (5R,6S)-2-(2-Hydroxyäthyl)-6-[(1R)-1-allyloxycarbonyloxyäthyl]-2-penem-3-carbonsäureallylester durch Umsetzung mit 95,2 mg β-N-Allyloxycarbonylalanin in die Titelverbindung überführt.
IR ($CH_2Cl_2$): 3440; 1790; 1730; 1245 $cm^{-1}$.
UV (Aethanol) $\lambda_{max}$ = 315 nm.

Beispiel 8: (5R,6S)-2-[2-(β-Alanyloxy)-äthyl]-6-[(1R)-1-hydroxyäthyl]-2-penem-3-carbonsäure

Analog Beispiel 6 werden 205 mg (5R,6S)-2-[2-(β-N-Allyloxycarbonylalanyloxy)-äthyl]-6-[(1R)-1-allyloxycarbonyloxyäthyl]-2-penem-3-car bonsäureallylester in die Titelverbindung überführt.
IR ($DMSO-d_6$): 3440; 1773; 1738; 1605; 1582 $cm^{-1}$.
UV (Wasser) $\lambda_{max}$ = 304 nm.

Beispiel 9:
(5R,6S)-2-{2-[2-(N-Allyloxycarbonylamino)-2-methyl-propionyloxy]-äthyl}-6-[(1R)-1-allyloxycarbonyloxyäthyl]-2-penem-3-carbonsäureallylester

Analog Beispiel 3 werden 191 mg (5R,6S)-2-(2-Hydroxyäthyl)-6-[(1R)-1-allyloxycarbonyloxyäthyl]-2-penem-3-carbonsäureallylester mit 103 mg 2-(N-Allyloxycarbonylamino)-2-methyl-propionsäure zur Titelverbindung umgesetzt.
IR ($CH_2Cl_2$): 3440; 1792; 1745 $cm^{-1}$.
UV (Aethanol) $\lambda_{max}$ = 313 nm.

Beispiel 10:
(5R,6S)-2-[2-(2-Amino-2-methyl-propionyloxy)-äthyl]-6-[(1R)-1-hydroxyäthyl]-2-penem-3-carbonsäure

Analog Beispiel 6 werden 206 mg (5R,6S)-2-{2-[2-(N-Allyloxycarbonylamino)-2-methyl-propionyloxy]-äthyl}-6-[(1R)-1-allyloxycarbonyloxyäthyl]-2-penem-3-carbonsäureallylester in die Titelverbindung überführt.
IR ($DMSO-d_6$): 3300; 1775; 1745; 1612; 1585 $cm^{-1}$.
UV (Wasser) $\lambda_{max}$ = 303 nm.

Beispiel 11:
(5R,6S)-2-{2-[3-(N-Allyloxycarbonylamino)-3-methyl-butyryloxy]-äthyl}-6-[(IR)-1-allyloxycarbonyloxyäthyl]-2-penem-3-carbonsäureallylester

Analog Beispiel 3 werden 191 mg (5R,6S)-2-(2-Hydroxyäthyl)-6-[(1R)-1-allyloxycarbonyloxyäthyl]-2-penem-3-carbonsäureallylester mit 110,6 mg 3-(N-Allyloxycarbonylamino)-3-methyl-buttersäure zur Titelverbindung umgesetzt.
IR ($CH_2Cl_2$): 3435, 1791, 1725 $cm^{-1}$.
UV (Aethanol) $\lambda_{max}$ = 315 nm.

Beispiel 12:
(5R,6S)-2-[2-(3-Amino-3-methyl-butyryloxy)-äthyl]-6-[(1R)-1-hydroxyäthyl]-2-penem-3-carbonsäure

Analog Beispiel 6 werden 243 mg (5R,6S)-2-{2-[3-N-Allyloxycarbonylamino)-3-methyl-butyryloxy]-äthyl}-6-[(1R)-1-allyloxycarbonyloxyäthyl]-2-penem-3-carbonsäureallylester in die Titelverbindung überführt.
IR ($DMSO-d_6$): 3430; 1775; 1735; 1610; 1585 $cm^{-1}$.
UV (Wasser) $\lambda_{max}$ = 305 nm.

Beispiel 13:
(5R,6S)-2-{2-[4-(N-Allyloxycarbonylamino)-4-methyl-pentanoyloxy]-äthyl}-6-[(1R)-1-allyloxycarbonyloxyäthyl]-2-penem-3-carbonsäureallylester

Analog Beispiel 3 werden 191 mg (5R,6S)-2-(2-Hydroxyäthyl)-6-[(1R)-1-allyloxycarbonyloxyäthyl]-2-penem-3-carbonsäureallylester mit 118,4 mg 4-(N-Allyloxycarbonylamino)-4-methyl-valeriansäure zur Titelverbindung umgesetzt.
IR ($CH_2Cl_2$): 3430; 1790; 1725 $cm^{-1}$.
UV (Aethanol) $\lambda_{max}$ = 315 nm.

Beispiel 14:
(5R,6S)-2-[2-(4-Amino-4-methyl-pentanoyloxy)-äthyl]-6-[(1R)-1-hydroxyäthyl]-2-penem-3-carbonsäure

Analog Beispiel 6 werden 253 mg (5R,6S)-2-{2-[4-(N-Allyloxycarbonylamino)-4-methyl-pentanoyloxy]-äthyl}-6-[(lR)-1-allyloxycarbonyloxyäthyl]-2-penem-3-carbonsäureallylester in die Titelverbindung überführt.
IR (DMSO-$d_6$): 3300; 1771; 1730; 1603; 1583 $cm^{-1}$.
UV (Wasser) $\lambda_{max}$ = 303 nm.

Beispiel 15: (5R,6S)-2-(2-Hydroxyäthyl)-6-[(1R)-1-hydroxyäthyl]-2-penem-3-carbonsäure, Natriumsalz

Analog Beispiel 6 werden 152,8 mg (5R,6S)-2-(2-Hydroxyäthyl)-6-[(1R)-1-allyloxycarbonyloxyäthyl]-2-penem-3-carbonsäureallylester in die Titelverbindung überführt.
IR (DMSO-$d_6$): 3430; 1770; 1610; 1582 $cm^{-1}$.
UV (Wasser) $\lambda_{max}$ 303 nm.

Beispiel 16:
(5R,6S)-2-(2-Carbamoyloxyäthyl)-6-[(1R)-1-allyloxycarbonyloxyäthyl]-2-penem-3-carbonsäureallylester

In 3,75 ml Methylenchlorid werden bei Raumtemperatur 357 mg (5R,6S)-2-(2-Hydroxyäthyl)-6-[(1R)-1-allyloxycarbonyloxyäthyl]-2-penem-3-carbonsäureallylester gelöst und mit 111,2 µl Trichloracetylisocyanat versetzt. Es wird 2 Stunden bei obiger Temperatur gerührt und dann nochmals 12,8 µl Trichloracetylisocyanat zugegeben. Nach 4 Stunden wird aufgearbeitet, indem mit Methylendichlorid das Reaktionsgemisch verdünnt, die organische Phase einmal mit Sole extrahiert und anschliessend über Natriumsulfat getrocknet wird. Die Lösung wird eingedampft, der Rückstand wird in 13,5 ml Methanol gelöst und mit 5,6 g Kieselgel 60 unter Stickstoffatmosphäre bei Raumtemperatur 5 Stunden gerührt. Dann werden nochmals 2 g Kieselgel 60 und 2 ml Methanol zugegeben und weitere 2 Stunden gerührt. Zur Aufarbeitung wird über Hyflo filtriert, mit Methanol nachgewaschen, das Filtrat am Vakuum eingeengt und der Rückstand an 20 g Kieselgel chromatographisch mit Toluol/Aethylacetat 4:1 gereinigt, wobei die obige Titelverbindung erhalten wird.
IR ($CH_2Cl_2$): 3540; 3420; 1790; 1735; 1710 $cm^{-1}$.
UV (Aethanol) $\lambda_{max}$ = 315 nm.

Beispiel 17: (5R,6S)-2-(2-Carbamoyloxyäthyl)-6-[(1R)-1-hydroxyäthyl]-2-penem-3-carbonsäure Natriumsalz

Analog Beispiel 6 werden 74 mg (5R,6S)-2-(2-Carbamoyloxyäthyl)-6-[(1R)-1-allyloxycarbonyloxyäthyl]-2-penem-3-carbonsäureallylester in die Titelverbindung überführt.
IR (DMSO-$d_6$): 3350; 3290; 3190; 1770; 1723; 1613; 1585 $cm^{-1}$.
UV (Wasser) $\lambda_{max}$ = 302 nm.

Beispiel 18:
(5R,6S)-2-[3-(2,2,2-Trichloräthoxycarbonyloxy)-propyl]-6-[(1R)-1-allyloxycarbonyloxyäthyl]-2-penem-3-carbonsäureallylester.

Eine Lösung von 183 g 2-[(3S,4R)-3-[(1R)-1-Allyloxycarbonyloxyäthyl]-4-[4-(2,2,2-trichloräthoxyarbonyloxy)-butyroylthio]-2-oxoazetidin-1-yl]-2-triäthoxyphosphoranylidenessigsäureallylester in 1,6, l abs. Xylol wird unter Stickstoffatmosphäre 1,5 Stunden bei Rückflusstemperatur gerührt. Dann wird das Lösungsmittel abgedampft und der Rückstand in 1 l Aethylacetat gelöst und dreimal mit 0,5 l Wasser gewaschen. Die wässrigen Extrakte werden zweimal mit 0,5 l Aethylacetat extrahiert. Die vereinigten organischen Lösungen werden über $MgSO_4$ getrocknet und eingedampft. Das Rohprodukt wird durch Chromatographie an Silicagel gereinigt (Laufmittel Toluol/Aethylacetat 9:1).
DC ($SiO_2$; Toluol/Aethylacetat 3:1) $R_f$=0,6.
IR (Methylenchlorid): 1790, 1750, 1705, 1580 $cm^{-1}$.
Das Ausgangsmaterial wird wie folgt hergestellt:

a)
(3S,4R)-3-[(1R)-1-Allyloxycarbonyloxyäthyl]-4-[4-(2,2,2-trichloräthoxycarbonyloxy)-butyroylthio]-azetidin-2-on

Eine Lösung von 207 g 4-(2,2,2-Trichloräthoxycarbonyloxy)-thiobuttersäure in 0,7 l Aethylacetat wird bei 0-5° während 5 Minuten mit 0,7 l Toluol und 0,32 l 2N Natronlauge ausgeschüttelt. Nach Trennung wird die wässrige Phase mit 0,3 l Toluol-Aethylacetat gewaschen. Die wässrige Lösung wird mit 4N HCl auf pH 10 gestellt und innert 5 Minuten zu einer Lösung von 102 g (3S,4R)-3-[(1R)-1-Allyloxycarbonyloxyäthyl]-4-tert.butylsulfonyl-acetidinon in 1280 ml Toluol und 320 ml Acetonitril gegeben. Das Reaktionsgemisch wird 1 Stunde unter Stickstoff bei Raumtemperatur stark gerührt. Nach Abtrennen der organischen Phase wird die wässrige Schicht nochmals mit 2x500 ml Aethylacetat gewaschen. Die vereinten organischen Extrakte werden nacheinander mit 500 ml Wasser und 500 ml Sole gewaschen, über $MgSO_4$ getrocknet und zur Trockene eingedampft. Das Rohprodukt wird durch Chromatographie an Silicagel gereinigt. (Lauftmittel: Methylenchlorid bis Methylenchlorid/Aceton: 95/5).
DC($SiO_2$; Methylenchlorid/Aceton 9:1): $R_f$=0,62
IR (Methylenchlorid): 3403, 1782, 1695 $cm^{-1}$.

b)
2-[(3S,4R)-3-[(1R)-1-Allyloxycarbonyloxyäthyl]-4-[4-(2,2,2-trichloräthoxycarbonyloxy)-butyroylthio]-2-oxo-azetidin-1-yl]-2-triäthoxyphosphoranylidenessigsäureallylester.

Eine Lösung von 110 g (3S,4R)-3-[(1R)-1-Allyloxycarbonyloxyäthyl]-4-[4-(2,2,2-trichloräthoxycarbonyloxy)-butyroylthio]-azetidin-2-on in 1,1 l abs. Methylenchlorid wird auf -15° gekühlt und mit 28,5 ml Allyloxalylchlorid und 40,6 ml Hünigbase innert 10 Minuten versetzt. Nach 30 Minuten Rühren unter Stickstoff bei -15° wird das Reaktionsgemisch bei 0° nacheinander mit 1,5 l 0,1 N HCl, 1,5 l ges. $NaHCO_3$-Lsg. und 1,5 l Wasser gewaschen. Die organische Phase wird über $MgSO_4$ getrochnet und am Vakuum eingedampft. Das rohe Oxalimid-Zwischenprodukt [IR (Methylenchlorid): 1815, 1755, 1710 $cm^{-1}$] wird ohne zusätzliche Reinigung in 442 ml Triäthylphosphit gelöst und 2 Stunden bei Raumtemperatur unter Stickstoff gerührt. Nach Zugabe von 0,5 l Decan wird das Reaktionsgemisch am Hochvakum bei 50° eingedampft. Das Eindampfen mit Decan wird nochmals wiederholt. Man erhält in zufriedenstellender Reinheit die Titelverbindung.
DC ($SiO_2$; Toluol/Aethylacetat 3:1) $R_f$=0,2.
IR ($CH_2Cl_2$): 1765, 1695, 1640 $cm^{-1}$.

Beispiel 19:
(5R,6S)-2-(3-Hydroxypropyl)-6-[(1R)-1-allyloxycarbonyloxyäthyl]-2-penem-3-carbonsäureallylester.

Eine Lösung von 111 g (5R,6S)-2-[3-(2,2,2-Trichloräthoxycarbonyloxy)-propyl]-6-[(1R)-1-allyloxycarbonyloxyäthyl]-2-penem-3-carbonsäureallylester in 2,15 l abs. Tetrahydrofuran und 291 ml einer wässrigen 1M Kaliumdihydrogenphosphat-Lösung wird mit 195 g Zinkstaub versetzt und während 4 Stunden bei Raumtemperatur stark gerührt. Die Suspension wird über Hyflo filtriert und das Filtrat wird dann eingedampft. Der Rückstand wird in 1,2 l Aethylacetat gelöst und nacheinander zweimal mit 500 ml gesättigter wässriger $NaHCO_3$-Lösung und einmal mit 500 ml Sole gewaschen und über $MgSO_4$ getrocknet. Nach Abdampfen des Lösungsmittels wird die Substanz Chromatographie an Silicagel (Laufmittel: Toluol/Aethylacetat 9:1 bis 4:1) gereinigt. DC ($SiO_2$; Toluol/Aethylacetat 3:1) $R_f$ = 0,22.
IR (Methylenchlorid): 3617, 1790, 1747, 1708, 1649, 1580 $cm^{-1}$.

Beispiel 20:
(5R,6S)-2-[3-(3-(N-Allyloxycarbonylamino)-propionyloxy)-propyl]-6-[(1R)-1-allyloxycarbonyloxyäthyl]-2-penem-3-carbonsäureallylester.

Analog Beispiel 3 werden 400 mg 3-(N-Allyloxycarbonylamino)-propionsäure durch Umsetzung mit (5R,6S)-2-(3-Hydroxypropyl)-6-[(1R)-1-allyloxycarbonyloxyäthyl]-2-penem-3-carbonsäureallylester in die Titelverbindung überführt.
IR ($CH_2Cl_2$): 3445, 1790, 1730, 1580 $cm^{-1}$.

Beispiel 21:
(5R,6S)-2-[3-(4-(N-Allyloxycarbonylamino)-4-methylpentanoyloxy)-propyl]-6-[(1R)-1-allyloxycarbonyloxyäthyl]-2-penem-3-carbonsäureallylester.

Analog Beispiel 3 werden 400 mg 4-(N-Allyloxycarbonylamino)-4-methylpentansäure durch Umsetzung mit (5R,6S)-2-(3-Hydroxypropyl)-6-[(1R)-1-allyloxycarbonyloxyäthyl]-2-penem-3-carbonsäureallylester in die Titelverbindung überführt.
IR ($CH_2Cl_2$): 3430, 1790, 1730, 1585 $cm^{-1}$.

Beispiel 22:
(5R,6S)-2-(3-Carbamoyloxypropyl)-6-[(1R)-1-allyloxycarbonyloxyäthyl]-2-penem-3-carbonsäureallylester.

Analog Beispiel 16 werden 445 mg (5R,6S)-2-(3-Hydroxypropyl)-6-[(1R)-1-allyloxycarbonyloxyäthyl]-2-penem-3-carbonsäureallylester in die Titelverbindung überführt.
IR ($CH_2Cl_2$): 3530, 3420, 1790, 1730, 1585 $cm^{-1}$.

Beispiel 23: (5R,6S)-2-[3-(3-Aminopropionyloxy)-propyl]-6-[(1R)-1-hydroxyäthyl]-2-penem-3-carbonsäure.

Analog Beispiel 6 werden 500 mg (5R,6S)-2-[3-(3-(N-Allyloxycarbonylamino)-propionyloxy)-propyl]-6-[(1R)-1-allyloxycarbonyloxyäthyl]-2-penem-3-carbonsäureallylester in die Titelverbindung überführt.
IR (DMSO-$d_6$): 3435, 1772, 1733, 1580 $cm^{-1}$.

Beispiel 24:
(5R,6S)-2-[3-(4-Amino-4-methyl-pentanoyloxy)-propyl]-6-[(1R)-1-hydroxyäthyl]-2-penem-3-carbonsäure.

Analog Beispiel 6 werden 540 mg (5R,6S)-2-[3-(4-(N-Allyloxycarbonylamino)-4-methyl-pentanoyloxy)-propyl]-6-[(1R)-1-allyloxycarbonyloxyäthyl]-2-penem-3-carbonsäureallylester in die Titelverbindung überführt.
IR (DMSO-$d_6$): 3434, 1772, 1728, 1600 $cm^{-1}$.

Beispiel 25: (5R,6S)-2-(3-Carbamoyloxypropyl)-6-[(1R)-1-hydroxyäthyl]-2-penem-3-carbonsäure, Natriumsalz.

Analog Beispiel 6 werden 275 mg (5R,6S)-2-(3-Carbamoyloxypropyl)-6-[(1R)-1-allyloxycarbonyloxyäthyl]-2-penem-3-carbonsäureallylester in die Titelverbindung überführt.
IR (DMSO-$d_6$): 3352, 2968, 1768, 1721, 1613 $cm^{-1}$.

Beispiel 26: (5R,6S)-2-(3-Hydroxypropyl)-6-[(1R)-1-hydroxyäthyl]-2-penem-3-carbonsäure, Natriumsalz.

Analog Beispiel 6 werden 1,98 g (5R,6S)-2-(3-Hydroxypropyl)-6-[(1R)-1-allyloxycarbonyloxyäthyl]-2-penem-3-carbonsäureallylester in die Titelverbindung überführt.
IR (DMSO-$d_6$): 3305, 1769, 1610 $cm^{-1}$.

Beispiel 27:

In analoger Weise, wie in den vorangehenden Beispielen beschrieben, können die folgenden Verbindungen hergestellt werden:
(5R,6S)-6-Hydroxymethyl-2-(2-sarcosyloxyäthyl)-2-penem-3-carbonsäure,
UV (Wasser) $\lambda_{max}$ = 307 nm.
(5R,6S)-6-Hydroxymethyl-2-(2-glycyloxyäthyl)-2-penem-3-carbonsäure,
UV (Wasser) $\lambda_{max}$ = 307 nm.
(5R,6S)-6-Hydroxymethyl-2-[2-(2S)-alanyloxyäthyl]-2-penem-3-carbonsäure,
UV (Wasser) $\lambda_{max}$ = 308 nm.
(5R,6S)-6-Hydroxymethyl-2-[2-(2S)-prolyloxyäthyl]-2-penem-3-carbonsäure,
UV (Wasser) $\lambda_{max}$ = 308 nm,
(5R,6S)-2-[(3R,S)-3-Aminobutyryloxy]-propyl]-6-[(1R)-1-hydroxyäthyl]-2-penem-3-carbonsäure,
UV ($H_2O$) $\lambda_{max}$ 307 nm,
(5R,6S)-2-[2-(2S)-Valyloxyäthyl]-6-[(1R)-1-hydroxyäthyl]-2-penem-3-carbonsäure,
UV ($H_2O$) $\lambda_{max}$ 308 nm,
(5R,6S)-2-[3-(2-Amino-2-methyl-propionyloxy)-propyl]-6-[(1R)-1-hydroxyäthyl]-2-penem-3-carbonsäure,
UV ($H_2O$) $\lambda_{max}$ 307 nm,
(5R,6S)-2-(3-Sarcosyloxypropyl)-6-[(1R)-1-hydroxyäthyl]-2-penem-3-carbonsäure,
UV ($H_2O$) $\lambda_{max}$ 307 nm,

Beispiel 28:

Trockenampullen oder Vials, enthaltend 0,5 g (5R,6S)-2-(2-Glycyloxyäthyl)-6-[(1R)-1-hydroxyäthyl]-2-penem-3-carbonsäure als Wirksubstanz, werden wie folgt hergestellt:

Zusammensetzung (für 1 Ampulle oder Vial):
Wirksubstanz 0,5 g
Mannit 0,5 g
Eine sterile wässerige Lösung der Wirksubstanz und des Mannits wird unter aseptischen Bedingungen in 5 ml-Ampullen oder 5 ml-Vials der Gefriertrocknung unterworfen. Anschliessend werden die Ampullen bzw. Vials verschlossen und geprüft.

Beispiel 29:

Kapseln enthaltend 0,5 g (5R,6S)-2-(2-Glycyloxyäthyl)-6-[(1R)-1-hydroxyäthyl]-2-penem-3-carbonsäure werden wie folgt hergestellt:

Zusammensetzung (für 2000 Kapseln)
(5R,6S)-2-(2-Glycyloxyäthyl)-6-[(1R)-1-hydroxyäthyl]-2-penem-3-carbonsäure 1000 g
Polyvinylpyrrolidon 15 g
Maisstärke 115 g
Magnesiumstearat 20 g

Man befeuchtet die (5R,6S)-2-(2-Glycyloxyäthyl)-6-[(1R)-1-hydroxyäthyl]-2-penem-3-carbonsäure mit 300 ml einer Lösung des Polyvinylpyrrolidons in 95 %-igem Aethanol, treibt das Gemisch durch ein Sieb mit 3 mm Maschenweite und trocknet das Granulat unter vermindertem Druck bei 40-50°. Man siebt durch ein Sieb mit 0,8 mm Maschenweite, gibt die Maisstärke und das Magnesiumstearat zu, vermischt und füllt das Gemisch in Steckkapseln ab.

Anstelle des obengenannten Wirkstoffs kann auch dieselbe Menge eines anderen Wirkstoffs der vorangehenden Beispiele verwendet werden.

Beispiel 30:

30 Tabletten, enthaltend 250 mg (5R,6S)-2-(2-Glycyloxyäthyl)-6-[(1R)-1-hydroxyäthyl]-2-penem-3-carbonsäure werden wie folgt hergestellt:

| Zusammensetzung: (für 1 Tablette) | |
|---|---|
| (5R,6S)-2-(2-Glycyloxyäthyl)-6-[(1R)-1-hydroxyäthyl]-2-penem-3-carbonsäure | 250 mg |
| Mikrokristalline Cellulose | 80 mg |
| Natrium-carboxymethyl-stärke | 10 mg |
| Magnesiumstearat | 3 mg |
| Talk | 7 mg |
| | 350 mg |

Der Wirkstoff wird mit den Zusatzstoffen homogen vermischt und zu Tabletten gepresst. Zur Herstellung von Filmdragées werden die Tabletten je mit 1 mg wässrigem Lack überzogen. Anstelle von Natriumcarboxymethyl-stärke kann auch Natrium-carboxy-methylcellulose eingesetzt werden.

Anstelle des obengenannten Wirkstoffs kann auch dieselbe Menge eines anderen Wirkstoffs der vorangehenden Beispiele verwendet werden.

**Patentansprüche**

1. Verbindungen der Formel

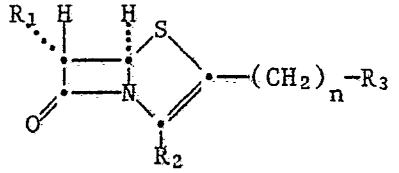

(I),

worin $R_1$ durch Hydroxy oder geschütztes Hydroxy substituiertes Niederalkyl ist, $R_2$ Carboxyl oder funktionell abgewandeltes Carboxyl bedeutet, n eine ganze Zahl von 2 bis 5 ist, $R_3$ Carbamoyloxy oder ein

Acyloxy-Rest der Formel $-O-C(=O)-Y-N(R_4,R_5)$ ist, worin $R_4$ Wasserstoff oder Niederalkyl ist, $R_5$ Wasserstoff, Niederalkyl oder Acyl ist und Y Niederalkylen, durch Phenyl substituiertes Niederalkylen oder ein Rest der Formel $-CR_6R_7$-ist, worin $R_6$ für einen über ein Kohlenstoffatom gebundenen organischen Rest steht oder zusammen mit $R_4$ gegebenenfalls durch Oxo oder Hydroxy substituiertes Niederalkylen bedeutet und $R_7$ Wasserstoff oder Niederalkyl bedeutet, die reinen optischen Isomere von Verbindungen der Formel (I), Mischungen dieser optischen Isomere und Salze davon.

2. Verbindungen der Formel I gemäss Anspruch 1, worin $R_1$ durch Hydroxy oder geschütztes Hydroxy substituiertes Niederalkyl ist; $R_2$ Carboxyl, unter physiologischen Bedingungen spaltbares verestertes Carboxyl oder geschütztes Carboxyl bedeutet; n eine ganze Zahl von 2 bis 5 ist, $R_3$ Carbamoyloxy oder ein Acyloxy-Rest der Formel $-O-C(=O)-Y-N(R_4R_5)$ ist, worin $R_4$ Wasserstoff oder Niederalkyl ist; $R_5$ Wasserstoff, Niederalkyl, Niederalkanoyl, durch Hydroxy, Niederalkoxy, Amino, Halogen, Carboxy und/oder Cyano substituiertes Niederalkanoyl; Benzoyl, durch Amino, Niederalkylamino, Diniederalkylamino, Niederalkanoylamino, Carboxy, Halogen, Nitro, Cyano und/oder Niederalkyl substituiertes Benzoyl, Phenylniederalkanoyl, im Niederalkanoylteil durch Amino oder Hydroxy substituiertes Phenylniederalkanoyl, Carbamoyl, Sulfo oder eine Aminoschutzgruppe ist und Y geradkettiges Niederalkylen mit 1 bis 7 Kohlenstoffatomen, verzweigtes Niederalkylen mit 2 bis 7 Kohlenstoffatomen, durch Phenyl substituiertes Niederalkylen oder eine Gruppe der Formel $-CR_6R_7-$ ist, worin $R_6$ Niederalkyl, Niederalkenyl, Cycloalkyl, Cycloalkenyl, Cycloalkadienyl, Phenyl, Phenylniederalkyl, einen über ein Ringkohlenstoffatom gebundenen monocyclischen 5-oder 6-gliedrigen gegebenenfalls partiell gesättigten Heteroarylrest mit 1-4 Ringstickstoffatomen und gegebenenfalls einem zusätzlichen Ringheteroatom ausgewählt aus der Gruppe Sauerstoff und Schwefel, z.B. einen entsprechenden aza-, diaza-, triaza-, tetraza-, oxa-, oxaza-, oxadiaza-, oxatriaza-, thia-, thiaza-, thiadiaza- oder thiatriaza-cyclischen Rest aromatischen Charakters, oder einen entsprechenden Dihydro- oder Tetrahydrorest, oder ein gegebenenfalls partiell gesättigtes Benzo-, Pyrido-oder Pyrimido-Derivat eines solchen 5-oder 6-gliedrigen Restes, oder einen durch einen über ein Ringkohlenstoffatom gebundenen monocyclischen 5- oder 6-gliedrigen gegebenenfalls partiell gesättigten Heteroarylrest mit 1-4 Ringstickstoffatomen und gegebenenfalls einem zusätzlichen Ringheteroatom ausgewählt aus der Gruppe Sauerstoff und Schwefel, oder einen durch einen über ein Ringstickstoffatom gebundenen monocyclischen, 5- oder 6-gliedrigen gegebenenfalls partiell gesättigten Heteroarylrest mit 1-4 Ringstickstoffatomen substituierten Niederalkylrest darstellt, welche Reste $R_6$ unsubstituiert oder durch Hydroxy, Niederalkoxy, Niederalkanoyloxy, Halogen, Mercapto, Niederalkylthio, gegebenenfalls durch Hydroxy, Niederalkoxy, Carboxy, Niederalkoxycarbonyl, Carbamoyl, gegebenenfalls N-niederalkyliertes Amino, Niederalkylenamino, Niederalkanoylamino, gegebenenfalls durch Amino und/oder Carboxy substituiertes Niederalkoxycarbonylamino, Mercapto, Niederalkylthio oder Sulfo substituiertes Niederalkyl; Amino, Niederalkylamino, Diniederalkylamino, Niederalkylenamino, Guanidino, Ureido, Niederalkanoylamino, gegebenenfalls durch Amino und/oder Carboxy substituiertes Niederalkoxycarbonylamino, Carboxyl, Niederalkoxycarbonyl, Carbamoyl, N-mono-oder N,N-diniederalkyliertes Carbamoyl, Cyano, Sulfo, Sulfamoyl, gegebenenfalls durch Niederalkyl, Nitro, Amino, Hydroxy, Niederalkoxy, Carboxy und/oder Halogen substituiertes Phenyl, Nitro, Oxo und/oder Oxido substituiert sind; oder worin $R_6$ zusammen mit $R_4$ gegebenenfalls durch Hydroxy oder Oxo substituiertes Niederalkylen bedeutet, und $R_7$ Wasserstoff oder Niederalkyl bedeutet, die reinen optischen Isomere von Verbindungen der Formel I, welche im Rest $R_1$ und/oder $R_3$ Chiralitätszentren besitzen, Mischungen dieser optischen Isomere und Salze davon.

3. Verbindungen der Formel I gemäss Anspruch 1, worin $R_1$ Hydroxymethyl oder 1-Hydroxyäthyl ist, $R_2$ Carboxyl oder eine unter physiologischen Bedingungen spaltbare veresterte Carboxylgruppe ist, n 2 oder 3 ist, und $R_3$ für Carbamoyloxy oder für den Acyloxyrest einer genetisch enkodierten Aminosäure oder für den Acyloxyrest von β-Alanin, 2-, 3- oder 4-Aminobuttersäure, α- oder β-Aminoisobuttersäure, 3-Amino-3-methyl-buttersäure, 4-Amino-4-methyl-pentansäure, 4-Aminopentansäure, oder für ein N-Niederalkyl-Derivat eines solchen Acyloxyrestes steht, die reinen Diastereomeren von Verbindungen der Formel (I), die im Rest $R_1$ und/oder $R_3$ Chiralitätszentren besitzen, Mischungen solcher Diastereomere, und Salze davon.

4. Verbindungen der Formel I gemäss Anspruch 1, worin $R_1$ Hydroxymethyl oder 1-Hydroxyäthyl ist, $R_2$ Carboxyl oder unter physiologischen Bedingungen spaltbares verestertes Carboxyl bedeutet, n 2 oder 3 ist und $R_3$ für Carbamoyloxy, Glycyloxy, Alanyloxy, β-Alanyloxy, 3-Amino-3-methylbutyryloxy oder 4-Aminobutyryloxy steht, die reinen Diastereomeren von Verbindungen der Formel (I), worin $R_1$ 1-Hydroxyäthyl ist und/oder die im Rest $R_3$ ein Chiralitätszentrum besitzen, Mischungen solcher Diastereomere, und Salze davon.

5. Verbindungen der Formel I gemäss Anspruch 1, worin $R_1$ 1-Hydroxyäthyl ist, $R_2$ Carboxyl bedeutet, n 2 ist und $R_3$ für Glycyloxy, β-Alanyloxy, Carbamoyloxy oder 4-Aminobutyryloxy steht, und Salze davon.

6. (5R,6S)-2-(2-Glycyloxyäthyl)-6-[(1R)-1-hydroxyäthyl]-2-penem-3-carbonsäure gemäss Anspruch 1.

7. (5R,6S)-2-[2-(β-Alanyloxy)-äthyl]-6-[(1R)-1-hydroxyäthyl]-2-penem-3-carbonsäure gemäss Anspruch 1.

8. (5R,6S)-2-[2-(4-Amino-butyryloxy)-äthyl]-6-[(1R)-1-hydroxyäthyl-2-penem-3-carbonsäure gemäss Anspruch 1.

9. (5R,6S)-2-(2-Carbamoyloxyäthyl)-6-[(1R)-1-hydroxyäthyl]-2-penem-3-carbonsäure und pharmazeutisch annehmbare Salze davon gemäss Anspruch 1.

10. (5R,6S)-2-[3-(3-Aminopropionyloxy)-propyl]-6-[(1R)-1-hydroxyäthyl-2-penem-3-carbonsäure gemäss Anspruch 1.

11. (5R,6S)-2-(3-Carbamoyloxypropyl)-6-[(1R)-1-hydroxyäthyl]-2-penem-3-carbonsäure und pharmazeutisch annehmbare Salze davon gemäss Anspruch 1.

12. Pharmazeutisches Präparat enthaltend eine Verbindung der Formel I gemäss Anspruch 1, wobei funktionelle Gruppen in ungeschützter Form vorliegen.

13. Eine Verbindung der Formel I gemäss Anspruch 1, worin funktionelle Gruppen in ungeschützter Form vorliegen, zur Anwendung in einem Verfahren zur therapeutischen Behandlung des menschlichen und trierischen Körpers.

14. Verwendung einer Verbindung der Formel I gemäss Anspruch 1, worin funktionelle Gruppen in ungeschützter Form vorliegen, zur Herstellung von pharmazeutischen Präparaten.

15. Verfahren zur Herstellung von Verbindungen der Formel I gemäss Anspruch 1, dadurch gekennzeichnet, dass man

a) eine Ylid-Verbindung der Formel

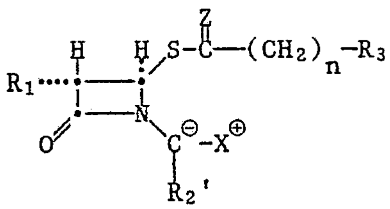

(II),

worin $R_1$, n und $R_3$ die unter Formel I angegebenen Bedeutungen haben, $R_2'$ eine geschützte Carboxylgruppe ist, Z Sauerstoff oder Schwefel bedeutet und $X^{\oplus}$ entweder eine dreifach substituierte Phosphoniogruppe oder eine zweifach veresterte Phosphonogruppe zusammen mit einem Kation darstellt, ringschliesst, oder

b) eine Verbindung der Formel

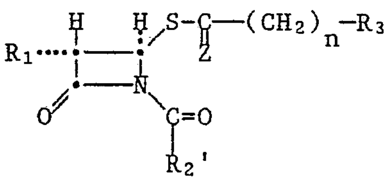

(III),

worin $R_1$, n und $R_3$ die unter Formel I angegebenen Bedeutung haben, Z Sauerstoff oder Schwefel ist und $R_2'$ eine geschützte Carboxylgruppe ist, mit einer organischen Verbindung des dreiwertigen Phosphors behandelt, oder

c) in einer Verbindung der Formel

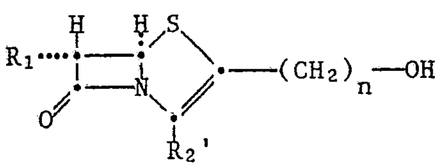

(IA) ,

worin $R_1$ und n die unter Formel I angegebenen Bedeutungen haben und $R_2'$ eine geschützte Carboxylgruppe ist, die Hydroxygruppe durch Acylierung in einen Acyloxyrest $R_3$ überführt, und wenn erwünscht oder notwendig, in einer erhältlichen Verbindung der Formel I eine geschützte funktionelle Gruppe in die freie funktionelle Gruppe überführt, und/oder, wenn erwünscht, in einer erhältlichen Verbindung der Formel I eine frei Carboxylgruppe $R_2$ in eine unter physiologischen Bedingungen spaltbare veresterte Carboxylgruppe überführt, und/oder, wenn erwünscht, in einer erhältlichen Verbindung der Formel I einen Rest $R_3$ in einen anderen Rest $R_3$ überführt, und/oder, wenn erwünscht, ein erhältliches Gemisch von isomeren Verbindungen der Formel I in die einzelnen Isomere auftrennt, und/oder, wenn erwünscht, eine erhältliche Verbindung mit salzbildender Gruppe in ein Salz oder ein erhältliches Salz in die freie Verbindung oder in ein anderes Salz überführt.

16. Die nach dem Verfahren gemäss Anspruch 15 erhältlichen Verbindungen.

**Patentansprüche für die folgenden Vertragstaaten: ES, GR**

1. Verfahren zur Herstellung von Verbindungen der Formel

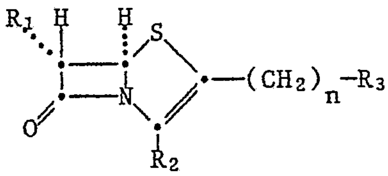

(I),

worin $R_1$ durch Hydroxy oder geschütztes Hydroxy substituiertes Niederalkyl ist, $R_2$ Carboxyl oder funktionell abgewandeltes Carboxyl bedeutet, n eine ganze Zahl von 2 bis 5 ist, $R_3$ Carbamoyloxy oder ein Acyloxy-Rest der Formel $-O-C(=O)-Y-N(R_4,R_5)$ ist, worin $R_4$ Wasserstoff oder Niederalkylist, $R_5$ Wasserstoff, Niederalkyl oder Acyl ist und Y Niederalkylen, durch Phenyl substituiertes Niederalkylen oder ein Rest der Formel $-CR_6R_7-$ ist, worin $R_6$ für einen über ein Kohlenstoffatom gebundenen organischen Rest steht oder zusammen mit $R_4$ gegebenenfalls durch Oxo oder Hydroxy substituiertes Niederalkylen bedeutet und $R_7$ Wasserstoff oder Niederalkyl bedeutet, den reinen optischen Isomeren davon, Mischungen dieser optischen Isomeren und Salzen davon,
dadurch gekennzeichnet, dass man

a) eine Ylid-Verbindung der Formel

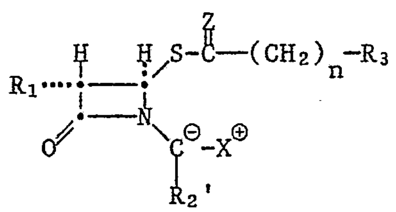

(II),

worin $R_1$, n und $R_3$ die unter Formel I angegebenen Bedeutungen haben, $R_2'$ eine geschützte Carboxylgruppe ist, Z Sauerstoff oder Schwefel bedeutet und $X^{\oplus}$ entweder eine dreifach substituierte Phosphoniogruppe oder eine zweifach veresterte Phosphonogruppe zusammen mit einem Kation darstellt, ringschliesst, oder

b) eine Verbindung der Formel

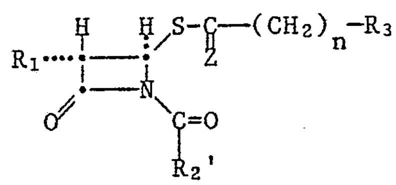

(III),

worin $R_1$, n und $R_3$ die unter Formel I angegebenen Bedeutungen haben, Z Sauerstoff oder Schwefel ist und $R_2'$ eine geschützte Carboxylgruppe ist, mit einer organischen Verbindung des dreiwertigen Phosphors behandelt, oderz

c) in einer Verbindung der Formel

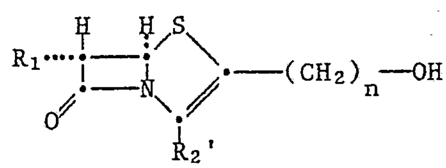

(IA) ,

worin $R_1$ und n die unter Formel I angegebenen Bedeutungen haben und $R_2'$ eine geschützte Carboxylgruppe ist, die Hydroxygruppe durch Acylierung in einen Acyloxyrest $R_3$ überführt, und wenn erwünscht oder notwendig, in einer erhältlichen Verbindung der Formel I eine geschützte funktionelle Gruppe in die freie funktionelle Gruppe überführt, und/oder, wenn erwünscht, in einer

erhältlichen Verbindung der Formel I eine frei Carboxylgruppe $R_2$ in eine unter physiologischen Bedingungen spaltbare veresterte Carboxylgruppe überführt, und/oder, wenn erwünscht, in einer erhältlichen Verbindung der Formel I einen Rest $R_3$ in einen anderen Rest $R_3$ überführt und/oder, wenn erwünscht, ein erhältliches Gemisch von isomeren Verbindungen der Formel I in die einzelnen Isomere auftrennt, und/oder, wenn erwünscht, eine erhältliche Verbindung mit salzbildender Gruppe in ein Salz oder ein erhältliches Salz in die freie Verbindung oder in ein anderes Salz überführt.

2. Verfahren zur Herstellung von Verbindungen der Formel I gemäss Anspruch 1, worin $R_1$ durch Hydroxy oder geschütztes Hydroxy substituiertes Niederalkyl ist; $R_2$ Carboxyl, unter physiologischen Bedingungen spaltbares verestertes Carboxyl oder geschütztes Carboxyl bedeutet; n eine ganze Zahl von 2 bis 5 ist, $R_3$ Carbamoyloxy oder ein Acyloxy-Rest der Formel $-O-C(=O)-Y-N(R_4,R_5)$ ist, worin $R_4$ Wasserstoff oder Niederalkyl ist; $R_5$ Wasserstoff, Niederalkyl, Niederalkanoyl, durch Hydroxy, Niederalkoxy, Amino, Halogen, Carboxy und/oder Cyano substituiertes Niederalkanoyl; Benzoyl, durch Amino, Niederalkylamino, Diniederalkylamino, Niederalkanoylamino, Carboxy, Halogen, Nitro, Cyano und/oder Niederalkyl substituiertes Benzoly, Phenylniederalkanoyl, im Niederalkanoylteil durch Amino oder Hydroxy substituiertes Phenylniederalkanoyl, Carbamoyl, Sulfo oder eine Aminoschutzgruppe ist und Y geradkettiges Niederalkylen mit 1 bis 7 Kohlenstoffatomen, verzweigtes Niederalkylen mit 2 bis 7 Kohlenstoffatomen, durch Phenyl substituiertes Niederalkylen oder eine Gruppe der Formel $-CR_6R_7-$ ist, worin $R_6$ Niederalkyl, Niederalkenyl, Cycloalkyl, Cycloalkenyl, Cycloalkadienyl, Phenyl, Phenylniederalkyl, einen über ein Ringkohlenstoffatom gebundenen monocyclischen 5-oder 6-gliedrigen gegebenenfalls partiell gesättigten Heteroarylrest mit 1-4 Ringstickstoffatomen und gegebenenfalls einem zusätzlichen Ringheteroatom ausgewählt aus der Gruppe Sauerstoff und Schwefel, z.B. einen entsprechenden aza-, diaza-, triaza-, tetraza-, oxa-, oxaza-, oxadiaza-, oxatriaza-, thia-, thiaza-, thiadiaza- oder thiatriaza-cyclischen Rest aromatischen Charakters, oder einen entsprechenden Dihydro- oder Tetrahydrorest, oder ein gegebenenfalls partiell gesättigtes Benzo-, Pyrido-oder Pyrimido-Derivat eines solchen 5-oder 6-gliedrigen Restes, oder einen durch einen über ein Ringkohlenstoffatom gebundenen monocyclischen 5- oder 6-gliedrigen gegebenenfalls partiell gesättigten Heteroarylrest mit 1-4 Ringstickstoffatomen und gegebenenfalls einem zusätzlichen Ringheteroatom ausgewählt aus der Gruppe Sauerstoff und Schwefel, oder einen durch einen über ein Ringstickstoffatom gebundenen monocyclischen, 5- oder 6-gliedrigen gegebenenfalls partiell gesättigten Heteroarylrest mit 1-4 Ringstickoffatomen substituierten Niederalkylrest darstellt, welche Reste $R_6$ unsubstituiert oder durch Hydroxy, Niederalkoxy, Niederalkanoyloxy, Halogen, Mercapto, Niederalkylthio, gegebenenfalls durch Hydroxy, Niederalkoxy, Carboxy, Niederalkoxycarbonyl, Carbamoyl, gegebenenfalls N-niederalkyliertes Amino, Niederalkylenamino, Niederalkanoylamino, gegebenenfalls durch Amino und/oder Carboxy substituiertes Niederalkoxycarbonylamino, Mercapto, Niederalkylthio oder Sulfo substituiertes Niederalkyl; Amino, Niederalkylamino, Diniederalkylamino, Niederalkylenamino, Guanidino, Ureido, Niederalkanoylamino, gegebenenfalls durch Amino und/oder Carboxy substituiertes Niederalkoxycarbonylamino, Carboxyl, Niederalkoxycarbonyl, Carbamoyl, N-mono-oder N,N-diniederalkyliertes Carbamoyl, Cyano, Sulfo, Sulfamoyl, gegebenenfalls durch Niederalkyl, Nitro, Amino, Hydroxy, Niederalkoxy, Carboxy und/oder Halogen substituiertes Phenyl, Nitro, Oxo und/oder Oxido substituiert sind; oder worin $R_6$ zusammen mit $R_4$ gegebenenfalls durch Hydroxy oder Oxo substituiertes Niederalkylen bedeutet, und $R_7$ Wasserstoff oder Niederalkyl bedeutet, den reinen optischen Isomeren, Mischungen dieser optischen Isomeren und Salzen davon.

3. Verfahren zur Herstellung von Verbindungen der Formel I gemäss Anspruch 1, worin $R_1$ Hydroxymethyl oder 1-Hydroxyäthyl ist, $R_2$ Carboxyl oder eine unter physiologischen Bedingungen spaltbare veresterte Carboxylgruppe ist, n 2 oder 3 ist, und $R_3$ für Carbamoyloxy oder für den Acyloxyrest einer genetisch enkodierten Aminosäure oder für den Acyloxyrest von β-Alanin, 2-, 3- oder 4-Aminobuttersäure, α-oder β-Aminoisobuttersäure, 3-Amino-3-methyl-buttersäure, 4-Amino-4-methylpentansäure, 4-Aminopentansäure, oder für ein N-Niederalkyl-Derivat eines solchen Acyloxyrestes steht, den reinen Diastereomeren von Verbindungen der Formel (I), die im Rest $R_1$ und/oder $R_3$ Chiralitätszentren besitzen, Mischungen solcher Diastereomeren, und Salzen davon.

4. Verfahren zur Herstellung von Verbindungen der Formel I gemäss Anspruch 1, worin $R_1$ Hydroxymethyl oder 1-Hydroxyäthyl ist, $R_2$ Carboxyl oder unter physiologischen Bedingungen spaltbares verestertes Carboxyl bedeutet, n 2 oder 3 ist und $R_3$ für Carbamoyloxy, Glycyloxy, Alanyloxy, β-Alanyloxy, 3-Amino-3-methyl-butyryloxy oder 4-Aminobutyryloxy steht, den reinen Diastereomeren von Verbindungen der Formel (I), worin $R_1$ 1-Hydroxyäthyl ist und/oder die im Rest $R_3$ ein Chiralitätszentrum besitzen, Mischungen solcher Diastereomere, und Salzen davon.

5. Verfahren zur Herstellung von Verbindungen der Formel I gemäss Anspruch 1, worin $R_1$ 1-Hydroxyäthyl ist, $R_2$ Carboxyl bedeutet, n 2 ist und $R_3$ für Glycyloxy, β-Alanyloxy, Carbamoyloxy oder 4-Aminobutyryloxy steht, und Salzen davon.

6. Verfahren gemäss Anspruch 1 zur Herstellung von (5R,6S)-2-(2-Glycyloxyäthyl)-6-[(1R)-1-hydroxyäthyl]-2-penem-3-carbonsäure.

7. Verfahren gemäss Anspruch 1 zur Herstellung von (5R,6S)-2-[2-(β-Alanyloxy)-äthyl]-6-[(1R)-1-hydroxyäthyl]-2-penem-3-carbonsäure.

8. Verfahren gemäss Anspruch 1 zur Herstellung von (5R,6S)-2-[2-(4-Amino-butyryloxy)-äthyl]-6-[(1R)-1-hydroxyäthyl]-2-penem-3-carbonsäure.

9. Verfahren gemäss Anspruch 1 zur Herstellung von (5R,6S)-2-[3-(3-Aminopropionyloxy)-propyl]-6-[(1R)-1-hydroxyäthyl-2-penem-3-carbonsäure.

10. Verfahren gemäss Anspruch 1 zur Herstellung von (5R,6S)-2-(2-Carbamoyloxyäthyl)-6-[(1R)-1-hydroxyäthyl]-2-penem-3-carbonsäure und pharmazeutisch annehmbarer Salze davon.

11. Verfahren gemäss Anspruch 1 Herstellung von (5R,6S)-2-(3-Carbamoyloxypropyl)-6-[(1R)-1-hydroxyäthyl]-2-penem-3-carbonsäure und pharmazeutisch annehmbarer Salze davon.

Europäisches Patentamt

# EUROPÄISCHER RECHERCHENBERICHT

Nummer der Anmeldung

EP 88 81 0407

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | EP-A-0 000 636 (GLAXO)<br>* Seiten 64-66, Beispiele 17-19; Ansprüche *<br>--- | 1,12,15 | C 07 D 499/00<br>A 61 K 31/43 //<br>C 07 F 7/18<br>C 07 F 9/65<br>C 07 D 205/08 |
| A | GB-A-2 042 514 (BRISTOL-MYERS)<br>* Zusammenfassung; Ansprüche *<br>--- | 1,12,15 | |
| P,A | EP-A-0 233 155 (CIBA-GEIGY)<br>* Ansprüche *<br>----- | 1,12,15 | |

| RECHERCHIERTE SACHGEBIETE (Int. Cl.4) |
|---|
| C 07 D 499/00<br>A 61 K 31/00<br>C 07 F 7/00 |

Der vorliegende Recherchenbericht wurde für alle Patentansprüche erstellt

| Recherchenort | Abschlußdatum der Recherche | Prüfer |
|---|---|---|
| DEN HAAG | 13-09-1988 | CHOULY J. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)